(19)

Europäisches
Patentamt
European
Patent Office
Office européen
des brevets

(11) **EP 2 741 669 B1**

(12) **EUROPEAN PATENT SPECIFICATION**

(45) Date of publication and mention
of the grant of the patent:
**18.04.2018 Bulletin 2018/16**

(21) Application number: **12783660.9**

(22) Date of filing: **14.08.2012**

(51) Int Cl.:
***A61B 5/11*** (2006.01)

(86) International application number:
**PCT/IB2012/054121**

(87) International publication number:
**WO 2013/030703 (07.03.2013 Gazette 2013/10)**

(54) **BED EXIT MONITORING APPARATUS**

ÜBERWACHUNGSVORRICHTUNG FÜR VERLASSEN DES BETTES

APPAREIL DE SURVEILLANCE DE SORTIE DE LIT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.09.2011 US 201161530549 P**
**25.10.2011 US 201161550957 P**

(43) Date of publication of application:
**18.06.2014 Bulletin 2014/25**

(73) Proprietors:
• **Koninklijke Philips N.V.**
**5656 AE Eindhoven (NL)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**
• **Philips Deutschland GmbH**
**20099 Hamburg (DE)**
Designated Contracting States:
**DE**

(72) Inventors:
• **TEN KATE, Warner Rudolph Theophile**
**NL-5656AA Eindhoven (NL)**
• **DURIC, Haris**
**NL-5656AA Eindhoven (NL)**
• **Van Den HEUVEL, Teun**
**NL-5656AA Eindhoven (NL)**
• **ESPINA PEREZ, Javier**
**NL-5656AA Eindhoven (NL)**

• **BINDSZUS, Andreas**
**NL-5656AA Eindhoven (NL)**
• **GREINER, Harald**
**NL-5656AA Eindhoven (NL)**

(74) Representative: **Steffen, Thomas**
**Philips Intellectual Property & Standards
High Tech Campus 5
5656 AE Eindhoven (NL)**

(56) References cited:
**EP-A1- 1 195 139      EP-A2- 1 115 350
WO-A1-2010/044013      WO-A1-2010/134010
WO-A2-2010/105045      DE-A1-102009 019 767
JP-A- 2011 172 885      US-A1- 2007 115 277
US-A1- 2008 190 202      US-A1- 2009 312 973**

• **VELTINK P H ET AL: "Procedure for in-use
calibration of triaxial accelerometers in medical
applications", SENSORS AND ACTUATORS A,
ELSEVIER SEQUOIA S.A., LAUSANNE, CH, vol.
68, no. 1-3, 15 June 1998 (1998-06-15), pages
221-228, XP004139834, ISSN: 0924-4247, DOI:
10.1016/S0924-4247(98)00049-1**
• **KARANTONIS D M ET AL: "Implementation of a
real-time human movement classifier using a
triaxial accelerometer for ambulatory
monitoring", IEEE TRANSACTIONS ON
INFORMATION TECHNOLOGY IN BIOMEDICINE,
IEEE SERVICE CENTER, LOS ALAMITOS, CA, US,
vol. 10, no. 1, 1 January 2006 (2006-01-01), pages
156-167, XP002593105, ISSN: 1089-7771, DOI:
10.1109/TITB.2005.856864**

**Description**

TECHNICAL FIELD OF THE INVENTION

The invention relates to an apparatus for monitoring a user to determine if they are about to get, or are getting, out of bed.

BACKGROUND TO THE INVENTION

Patient falls are among the most common occurrences reported in hospitals and are a leading cause of death in people of age 65 or older. Of those who fall, as many as half may suffer moderate to severe injuries that reduce mobility and independence, and increase the risk of premature death. About 50% of older adults hospitalized for hip fracture never regain their previous level of function. Around 10% of fatal falls by older adults occur in hospitals.

It is therefore a goal to reduce the risk of patient harm resulting from falls. Existing fall prevention strategies rely on identifying the patients that are at an increased risk of falls and using a device or system that is integrated into their bed to monitor the patient and to provide alarms to nursing staff at the point that the patient is exiting their bed. Particular types of patients that may require this monitoring include the elderly, young children and patients with specific conditions or circumstances, such as post-surgery.

It will be appreciated that these monitoring systems can also be applied to patients other than those deemed to be a fall risk, for example they can be used to alert nursing staff that a patient who requires assistance moving a trolley holding drips and other medical equipment that is attached to the patient is leaving their bed.

Current bed exit detection technologies that are used in hospitals often rely on pressure-sensitive mats or load cells integrated into the patient's bed. Other common types of sensors are based on infrared beam detectors, mounted in parallel to the side rails of the bed. Alternatively, ultrasound-based bed presence detectors can be used. Although these technologies may be effective in detecting that the patient has actually left the bed, the alarms provided by these technologies often come too late and do not provide enough time for the nursing staff to react and reach the patient to prevent a fall. Another common problem with these technologies is that they have a relatively high false alarm rate. In each case, dedicated equipment is required to be installed on or around the bed, which can be used only for detecting whether a user has got out of bed.

Therefore, there is need for an alternative bed exit monitoring system that can detect when the patient or user has got out of bed (and that can potentially detect that the patient or user is about to get out of bed), thereby providing an alarm to nursing staff as early as possible, whilst minimizing the occurrence of false alarms.

Some background information can be found in WO 2010/134010 and EP1115350.

SUMMARY OF THE INVENTION

According to a first aspect of the invention, there is provided a bed exit monitoring apparatus for monitoring a user and determining when the user has got out of a bed as defined in claim 1 of the appended claims.

In some embodiments, the processor can be further configured to receive measurements of the orientation of the device; and use the measurements of the orientation of the device with the measurements of acceleration to estimate the posture of the user over time.

The reference vectors can correspond respectively to: the z-axis of the reference frame of the user which is in the posterior-anterior direction, the y-axis of the reference frame of the user which is vertical from bottom to top, and the x-axis of the reference frame of the user which is in the medial to lateral direction; the measure of the correspondence in direction of the acceleration vectors and the reference vector for the z-axis of the reference frame of the user can indicate the correspondence of the measurements of acceleration to a prone or supine posture of the user; the measure of the correspondence in direction of the acceleration vectors and the reference vector for the y-axis of the reference frame of the user can indicate the correspondence of the measurements of acceleration to an upright posture of the user; and the measure of the correspondence in direction of the acceleration vectors and the reference vector for the x-axis of the reference frame of the user can indicate the correspondence of the measurements of acceleration to a posture in which the user is lying on their side.

In some embodiments, the processor is configured to determine a measure of the correspondence in direction of the acceleration vectors and a plurality of the reference vectors corresponding to axes of the reference frame of the user; and estimate the posture of the user for an acceleration vector based on the measure of the correspondence in direction that has the largest absolute value or magnitude.

According to a second aspect of the invention, there is provided a device that is configured to be worn by a user, the device comprising an accelerometer that measures the acceleration acting on the device in three-dimensions; and an apparatus as described above.

According to a third aspect of the invention, there is provided a bed exit monitoring system, the system comprising a device that is configured to be worn by a user, the device comprising an accelerometer that measures the acceleration acting on the device in three-dimensions; and a base unit that is configured to communicate with the device, and that comprises an apparatus as described above.

According to a fourth aspect of the invention, there is provided a method of monitoring a user and determining when the user has got out of a bed as defined in claim 6 of the appended claims.

In some embodiments, the method can further comprise the step of receiving measurements of the orientation of the device; and wherein the step of processing the measurements comprises using the measurements of the orientation of the device with the measurements of acceleration to estimate the posture of the user over time.

The reference vectors can correspond respectively to: the z-axis of the reference frame of the user which is in the posterior-anterior direction, the y-axis of the reference frame of the user which is vertical from bottom to top, and the x-axis of the reference frame of the user which is in the medial to lateral direction; the measure of the correspondence in direction of the acceleration vectors and the reference vector for the z-axis of the reference frame of the user can indicate the correspondence of the measurements of acceleration to a prone or supine posture of the user; the measure of the correspondence in direction of the acceleration vectors and the reference vector for the y-axis of the reference frame of the user can indicate the correspondence of the measurements of acceleration to an upright posture of the user; and the measure of the correspondence in direction of the acceleration vectors and the reference vector for the x-axis of the reference frame of the user can indicate the correspondence of the measurements of acceleration to a posture in which the user is lying on their side.

In some embodiments, the step of processing the measurements comprises determining a measure of the correspondence in direction of the acceleration vectors and a plurality of the reference vectors corresponding to axes of the reference frame of the user; and estimating the posture of the user for an acceleration vector based on the measure of the correspondence in direction that has the largest absolute value or magnitude.

According to a fifth aspect of the invention, there is provided a computer program product, comprising a computer readable medium having computer program code embodied therein, the computer program code being configured such that, upon execution by a computer or processor, the computer or processor performs the method as described above.

BRIEF DESCRIPTION OF THE DRAWINGS

Embodiments of the invention will now be described, by way of example only, with reference to the following drawings, in which:

Fig. 1 is a block diagram of a bed exit monitoring system according to an embodiment of the invention;
Fig. 2(a) is a graph illustrating the signals obtained by an accelerometer during a bed exit by a user and Fig. 2(b) is a graph illustrating the inclination of the user during the bed exit;
Fig. 3 is a graph illustrating the change in position of the user in a global coordinate system during a bed exit;
Fig. 4 is a graph illustrating the altitude or height change during a bed exit derived from measurements of changes in air pressure;
Fig. 5 is a flow chart illustrating a method of processing measurements of the movement of the user of the system in order to determine if the user is getting, or is about to get out of the bed;
Fig. 6 is a flow diagram illustrating a method of calibrating the device according to an embodiment of the invention;
Fig. 7 is a flow diagram illustrating a method of estimating the posture of the user from accelerometer measurements;
Fig. 8 is a pair of graphs illustrating measurements from an accelerometer and postures estimated from the accelerometer measurements;
Fig. 9 is a flow chart of a method for determining whether a detected height change and the estimated postures correspond to a bed exit;
Fig. 10 is a flow chart of an alternative method for determining whether a detected height change and the estimated postures correspond to a bed exit;
Fig. 11 is a block diagram illustrating the processing of an accelerometer signal to determine a height change;
Fig. 12 is a flow chart illustrating a method of processing an accelerometer signal to determine a height change;
Fig. 13 is a series of graphs showing the signals at various stages of the processing to determine a height change;
Fig. 14 is a set of graphs showing the change in height and height estimated from an exemplary set of acceleration measurements;
Fig. 15 is a block diagram of a subMedian filter;
Fig. 16 is a graph illustrating a basic principle of operation of the subMedian filter;
Fig. 17 is another graph illustrating the operation of the subMedian filter;
Fig. 18 is a flow chart illustrating an exemplary method of operating the subMedian filter;
Fig. 19 is a graph illustrating the result of applying a subMedian filter to a signal comprising a set of sinusoidal signals and Gaussian noise;

Fig. 20 is a graph illustrating the median filter subwindows used by an adaptive median filter; and

Fig. 21 is a block diagram of an exemplary adaptive median filter.

## DETAILED DESCRIPTION OF THE PREFERRED EMBODIMENTS

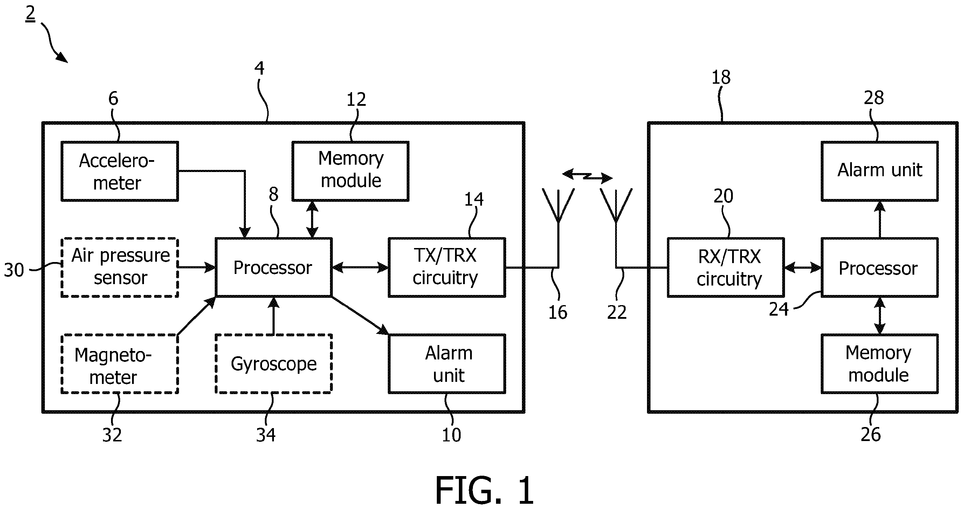

An embodiment of a bed exit monitoring apparatus in the form of a bed exit monitoring system 2 in accordance with the invention is shown in Fig. 1. The system 2 comprises a device 4 that is to be worn by a user. The device 4 can be provided in the form of a pendant with a neck cord for placement around the user's neck, but alternatively the device 4 can be configured to be worn at or on a different part of the user's body, such as the wrist, waist, trunk, thorax, pelvis or sternum, and will comprises a suitable arrangement for attaching the device 4 to that part of the body (for example a belt or strap).

The device 4 comprises a sensor 6 for measuring the accelerations acting on the device 4, which, assuming that the device 4 is being correctly worn by the user, correspond to the accelerations experienced by the user. This sensor 6, for example an accelerometer, outputs the acceleration measurements (signals) to a processor 8 in the device 4. In some embodiments, the accelerometer 6 is a micro-electromechanical system (MEMS) accelerometer. The acceleration experienced by the accelerometer 6 may be sampled at a rate of 30 Hz, although it will be appreciated that many other sampling frequencies can be used (for example 50 and 125 Hz).

The advantage of using a device 4 having an accelerometer 6 attached to the user is that the accelerometer signals can be processed in order to identify whether the user is performing movements that are consistent with the user getting out of bed, as well as the system 2 being able to use the accelerometer measurements to measure vital signs of the user, such as respiration and/or pulse rate. In addition, the presence of an accelerometer 6 in the device 4, with suitable processing, allows the device 4 to determine the condition or current status of the user (i.e. standing up, walking, fallen, etc.). This is in contrast to the conventional systems that make use of pressure-sensitive mats or infrared beam detectors which can only detect that the user gets out of the bed (i.e. they do not provide any other monitoring or detection functions). Furthermore, these conventional systems cannot provide any indication of the status or condition of the user once they have got out of bed. Lastly, they need to be installed by the hospital personnel for the purpose of bed exit detection, causing additional burden.

The processor 8 in the device 4 analyses or processes the measurements from the accelerometer 6 to determine if the user is getting, or is going to get, out of bed.

The device 4 further comprises an alarm unit 10 (which may comprise a loudspeaker) that can be triggered by the processor 8 if a bed exit is detected in order to summon help to the user. However, it will be appreciated that the presence of an alarm unit 10 in the device 4 is optional.

The device 4 further comprises a memory module 12 that is connected to the processor 8 and that can store the measurements from the accelerometer 6 before, during or after the processing of the measurements by the processor 8. The memory module 12 can also store the results of the processing performed by the processor 8. In addition, the memory module 12 may store computer code or program instructions relating to the processing steps to be performed by the processor 8 in order to determine if a bed exit is occurring or is about to occur, which can be retrieved and executed by the processor 8 as required.

The device 4 further comprises transmitter (TX) or transceiver (TRX) circuitry 16 and associated antenna 18 that can be used for transmitting the accelerometer measurements or the results of the processing (which can include an alarm signal) to a base unit 18. The base unit 18 may, for example, be located proximate to the bed of the user or it may, for example, be a computer terminal at a nurse station. In other embodiments, there may a base unit 18 located near to the bed of the user that receives the measurements or processing results from the device 4 and transmits the information to another unit, such as a computer terminal, located at a nurse station.

The base unit 18 comprises respective receiver (RX) or transceiver (TRX) circuitry 20 and antenna 22 for receiving transmissions (such as the accelerometer measurements, processing results and/or alarm signal) from the device 4 and a processor 24 for controlling the operation of the base unit 18. The base unit 18 may use the TRX circuitry 20 to transmit settings or configuration information to the device 4. Although not illustrated, the base unit 18 may also comprise a display for illustrating the results of the processing (for example a respiration and/or pulse rate).

The base unit 18 also optionally comprises a memory module 26 that is used for storing the information received from the device 4 along with computer code or program instructions relating to the processing steps to be performed by the processor 24 in order to control the operation of the base unit 18.

The base unit 28 also comprises an alarm unit (which may comprise a loudspeaker) that can be triggered by the processor 24 if a bed exit is detected.

Although in the embodiment of the invention described herein the processor 8 performs the processing of the accelerometer measurements to determine if the user is getting, or is going to get, out of bed, it will be appreciated that in an alternative embodiment of the invention, processor 8 in the device 4 can simply transmit the accelerometer measurements to the base unit 18 via the transceiver circuitry 14 and the processing of the accelerometer measurements to determine whether the user is getting, or is going to get, out of bed can be performed by the processor 24 in the base unit 18.

In a further alternative, the processor 8 in the device 4 may perform some of the initial processing steps on the accelerometer measurements before transmitting the results to the base unit 18 which, for example, completes the processing and determines whether the user is getting out of bed.

Briefly, the measurements from the accelerometer 6 are processed to in order to determine if the user is getting out of bed. As described in more detail below, this processing aims to identify movements and/or postures that are consistent with the user getting out of bed. For example, a typical bed exit will involve the user rolling onto their side so that they face the edge of the bed (assuming they start in a prone or supine position), sitting up, and then standing upright. Thus, the accelerometer measurements can be processed to identify any one or more of movements, movement patterns, changes in body posture, changes in orientation and/or changes in height that are consistent with the user getting out of bed. Although it is possible to process the measurements to identify just one posture or just a change in height to determine if the user is getting out of bed, it will be appreciated that the risk of false alarms being generated can be reduced by identifying multiple postures in conjunction with a change in height from the measurements. Furthermore, the risk of false alarms can be further reduced by determining whether the posture(s) and change in height occur in the correct temporal order for a bed exit.

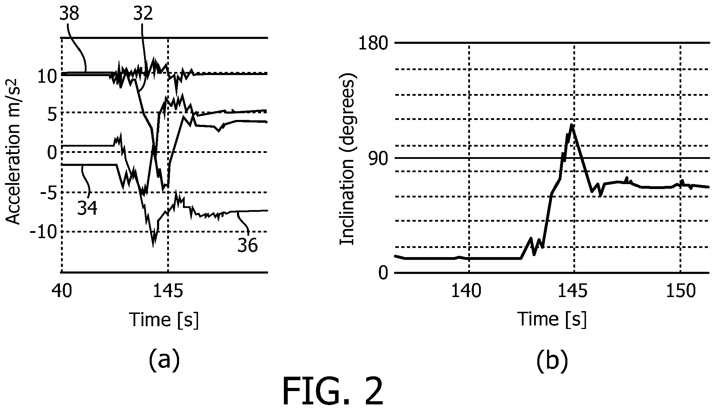

Figs. 2(a) and (b) show a typical example of signals from an accelerometer 6 when a person exits a bed. In Fig. 2(a) the user is initially lying in a supine position. Lines 32, 34 and 36 represent the measurements of acceleration along the z-, y- and x-axes of the accelerometer 6 respectively. The device 4 and accelerometer 6 are typically oriented such that the z-axis is aligned substantially normal to the user's body (i.e. it is perpendicular to the plane of the skin on the user's back directed towards the front of the user such that gravity is measured by this axis when the user is lying supine or prone), the y-axis is aligned substantially vertically upwards from the ground along the user's body, and the x-axis is orthogonal to the y- and z-axes. In biomechanics terms, the z-axis is in the posterior-anterior direction, the y-axis is vertical from bottom to top and the x-axis is in the medial to lateral direction (where the x-, y- and z-axes are orthogonal, right-handed. Line 38 represents the norm of the acceleration signal, which equals the size of gravity when no other accelerations are present. In this Figure, the orientation of the device 4 is such that components of acceleration corresponding to gravity are found along each of the x-, y- and z-axes.

Then, the user turns onto their side facing to bed edge and rises to stand on their feet. Fig. 2(b) shows the inclination, measured in degrees, which rises when the user bends towards the edge of the bed, and even assumes a forward bent posture before the user stands up. The end of the inclination signal corresponds to an upright posture. Ideally (i.e. if the accelerometer 6 is oriented correctly on the user, the inclination will be 90 degrees when the user is standing, in accordance with the gravity observed in the z-axis direction.

The posture sequence from lying to sitting upright (or sitting with forward inclination) is a feature that can be detected by the bed exit monitoring system 2. In another embodiment, the intermediate postural steps that happen in the posture transition can be analyzed to increase the reliability of the detection. If, for example, a movement of the trunk in both forward and lateral directions is simultaneously detected (possibly in addition to detecting a change in height of the trunk), this means that the subject is attempting to exit bed by pulling themselves towards the edge of the bed in a supine position and then hanging their legs out of the bed to gain floor contact.

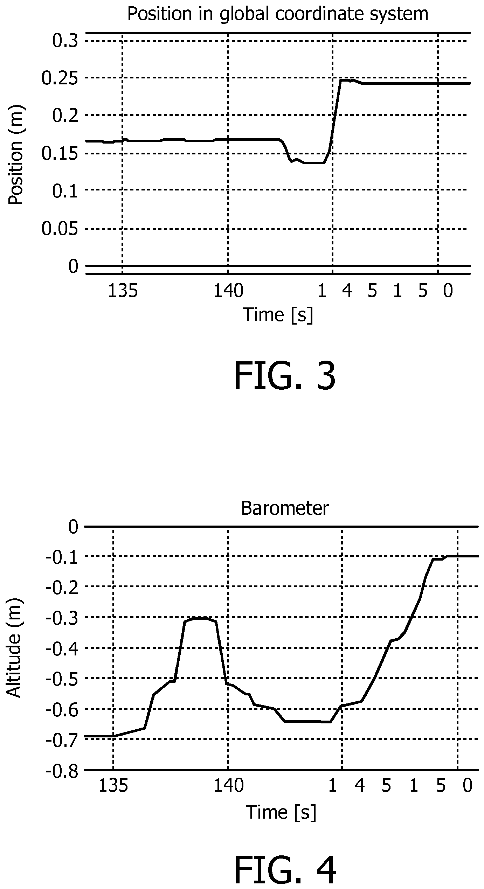

By using a filter to estimate the vertical component of the acceleration, for example by computing the norm of the acceleration signal, and to remove the acceleration corresponding to gravity from the accelerometer signals, the rise of the trunk - i.e. going from the lying or sitting position to standing upright after exiting the bed - can be measured by twice integrating the (filtered) accelerometer signal. This rise provides another feature that can be used in the detection of a bed exit. The height rise as computed from the acceleration signal shown in Fig. 2(a) is shown in Fig. 3.

In some embodiments, the device 4 can comprise one or more additional sensors 30, 32 and/or 34 that can provide measurements in addition to the accelerometer measurements, which can be used to supplement the processing of the accelerometer measurements and thereby allow the detection of further movements or postures and thus increase the reliability of the bed exit detection. According to the invention, the device 4 further comprises an air pressure sensor 30 that provides air pressure measurements that can be processed to determine the height or altitude of the device 4. Fig. 4 shows a signal representing altitude or height that is derived from an air pressure sensor signal obtained from air pressure sensor 30 during a bed exit.

Fig. 4 illustrates the desirability of combining the analysis of an air pressure sensor signal with an accelerometer measurement. In particular, this combination makes it possible to distinguish environmental changes in air pressure from those due to a physical change in height. On the other hand, when using the accelerometer signal to determine a height difference, the use of the air pressure sensor signals allows apparent height changes due to motion of the device 4 to be distinguished from actual height changes. For example, if the accelerometer 6 is no longer properly calibrated, a rotation of the device 4 may cause a change in the magnitude of the measured gravity, which can be interpreted as an acceleration due to movement and thus would lead to an apparent change in height.

Furthermore, as it is difficult to measure rotations around the vertical using an accelerometer 6, the device 4 may also or alternatively comprise a magnetometer 32 and/or gyroscope 34 which provide measurements that are useful for determining the orientation of the device 4 in a horizontal plane (i.e. determining the rotation or heading of the user around the vertical gravity field). Since sitting up on the edge of a bed edge typically also involves the user turning sideways, (i.e. the user performs a rotation around the vertical axis), the addition of a magnetometer 32 or gyroscope 34 can improve the bed exit detection.

In addition to detecting whether a user is about to get out of bed, it will be appreciated that it is possible for the sensor measurements to be processed to determine if the user has actually got out of bed. For example, the measurements can be processed to determine if the user has stood-up or is already standing or walking. Standing up can be determined upon detection of a given height increase and/or characteristic movement patterns (for example the swinging of the trunk back and forth just before a height increase). Standing may be determined upon detection of an upright trunk posture and a slight swaying (which makes it distinctive from sitting upright). Walking may be determined upon the simultaneous detection of upright trunk posture and an increased movement level (e.g. in terms of energy or variance in the accelerometer signals).

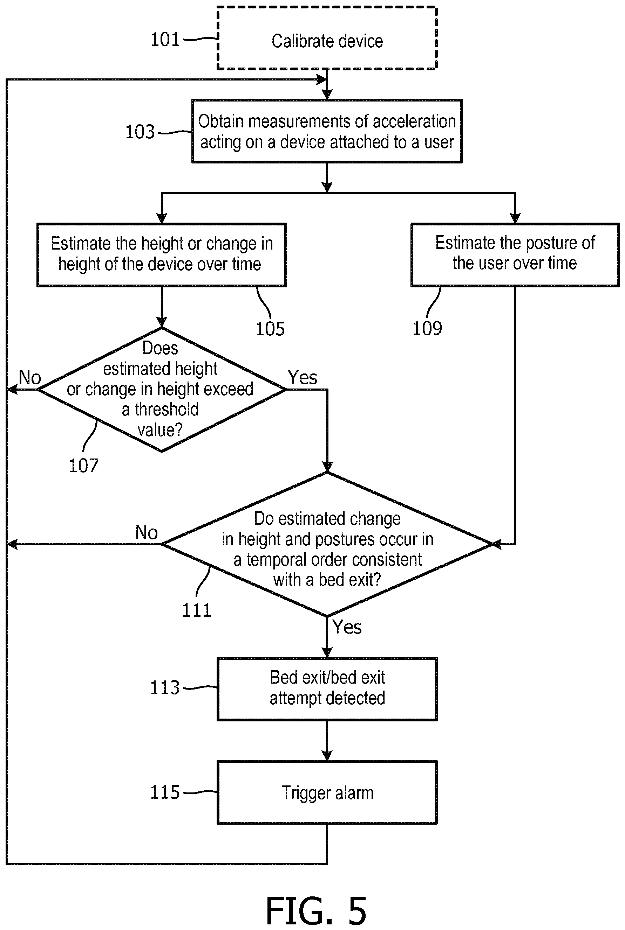

Fig. 5 is a flow chart illustrating some exemplary steps in a method according to an embodiment of the invention. In step 101 (which is optional), during or after attachment of a device 4 as described above to a user, the device 4 is calibrated. This calibration involves calibrating the device 4 so that the measurements from the accelerometer 6 can be converted into the reference frame of the user, thereby allowing the measurements to be used to more accurately determine the posture of the user. It will be appreciated that the calibration step may be performed periodically during use of the device 4, and may be performed in parallel with subsequent steps in the method.

In step 103, measurements of the acceleration acting on the device 4 are obtained from the accelerometer 6.

In the following steps of the method, as illustrated with reference to steps 105 to 111, the accelerometer measurements are processed in order to determine if the user is going to get, or is getting, out of bed. It will be appreciated that steps 105 to 111 can be performed in a different order to that illustrated and described below, and/or some of the steps may only be performed if the results of preceding steps are consistent with a bed exit having occurred.

In particular, in step 105 the height or change in height of the device 4 over time is estimated from the accelerometer measurements.

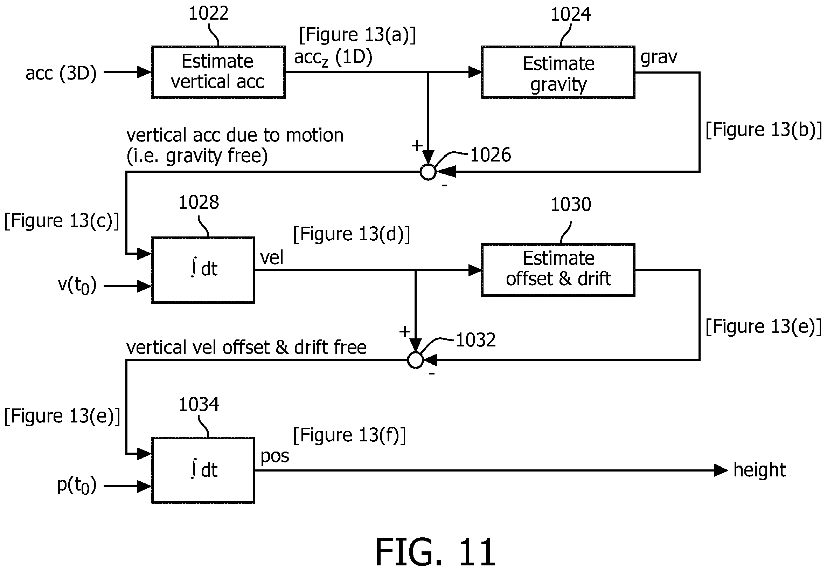

Where the device 4 does not include any additional sensors to the accelerometer 6, the height or change in height of the device 4 can be determined solely from the accelerometer measurements, for example as described International patent publication no. WO2013024461A1 entitled "Estimating velocity in a horizontal or vertical direction from acceleration measurements", which was filed on 18 August 2011 (U.S. Patent Application) in the name of Koninklijke Philips Electronics N.V. The technique for estimating velocity and height or a change in height described in that patent application is set out in the Appendix at the end of this description and illustrated in Figs. 11 to 21.

Alternatively, where the device 4 comprises a height sensor, such as an air pressure sensor 30, the air pressure sensor measurements can be used to determine the height or change in height instead of, or in conjunction with, the accelerometer measurements, for example by using sensor fusion techniques. Where the device 4 comprises a further sensor, such as a magnetometer 32 or gyroscope 34, the measurements from these sensors can be used in conjunction with the measurements from the accelerometer 6 to identify the vertical component of acceleration and thus assist in determining the height or change in height of the device 4.

The estimated height or change in height is then compared to a threshold value (step 107) to determine if the height or change in height is consistent with the user getting out of bed. The threshold value can be set based on various factors, including the position of the device 4 on the user, the height of the user, the height of the bed, etc. In some implementations, the threshold may be set corresponding proportionally to the difference in height of the waist when the user is lying down and when the user is standing upright. Thus, where the bed is quite high, and/or where the user is relatively short, the threshold will be set to a relatively low value. As an example, where the device 4 is attached to the waist of a user, a suitable threshold for a change in height could be 4 centimetres (0.04 metres).

If the estimated height or change in height exceeds the threshold value, the method passes to step 111. If the estimated height or change in height does not exceed the threshold value, the method returns to step 103 (i.e. subsequent steps are not executed for the current measurements) and repeats for a new set of acceleration measurements.

In addition to estimating the height change in step 105, the processor 8 estimates the posture for the user at a number of time instants (step 109).

As with the height change estimation above, the posture estimation can be accomplished using just the measurements from the accelerometer 6, which is described in more detail below. Alternatively, the postures can be estimated from the accelerometer measurements in conjunction with the measurements from a further sensor, such as a magnetometer 32 or gyroscope 34. The measurements from the magnetometer 32 or gyroscope 34 allow the estimation of the orientation of the device 4 to be improved.

In step 111, which, in this embodiment, occurs after a positive determination of a height or change in height above the threshold value, it is determined whether the estimated change in height and estimated postures are consistent with a bed exit. This step is described in more detail below. In one embodiment, it is determined whether the time at which the height exceeds the threshold occurs between the user being in a lying (supine, prone or side) position and being in an upright position. If not, then it is determined that the measured height change and movements do not correspond to the user getting out of bed and the method returns to step 103.

If the time at which the height exceeds the threshold or time period during which the change in height exceeds the threshold does occur during the transition from the lying position to the upright position, it is determined that a bed exit or attempt at a bed exit has occurred (step 113). In that case, an alarm can be triggered (step 115).

The processing performed in the steps of the method shown in Fig. 5 will now be described in more detail. In the following, it is assumed that the device 4 is attached to the waist of the user.

Calibration

As suggested above, as the precise orientation of the device 4 and hence accelerometer 6 with respect to the reference frame of the user is unknown, it is desirable to perform a calibration step that involves calibrating the device 4 so that the measurements from the accelerometer 6 can be converted into the reference frame of the user, thereby allowing the measurements to be used to more accurately determine the change in height of the device 4 and/or the posture of the user.

As described above, the form factor/shape of the device 4 (particularly the housing) can be designed such that upon attachment to, for example, the waist area of the user, the z-axis of the accelerometer 6 is aligned substantially normal to the user's body (i.e. it is perpendicular to the plane of the skin on the user's back), the y-axis is aligned substantially vertically down towards the ground through the user's body (i.e. it points towards the user's legs when the device 4 is attached to the waist), and the x-axis is orthogonal to the y- and z-axes (i.e. it points in a lateral direction). The device 4 may have markings on the housing of the device 4 indicating the preferred orientation when it is attached to the user, or it may be ergonomically-shaped for a particular part of the user's body so that the device 4 assumes substantially the correct orientation when attached to the user.

When the device 4 is substantially aligned with the reference frame of the user, the acceleration sensed in the y-direction should have the largest component of acceleration when the user is standing upright since gravity will act in that direction (assuming there is not much movement, otherwise the "DC" component will be largest along the y-direction). With the y-axis aligned downwards, the acceleration corresponding to gravity will have a positive sign when the user is standing upright. Similarly, when the user is supine (i.e. lying on their back), acceleration corresponding to gravity will result in the signal along the z-axis to be the largest (also with a positive sign). When the user is lying on their side, the signal along the x-axis will be largest (with the sign depending on whether the user is lying on their right or left side).

It may be possible to attach the device 4 to the user in such a way that the measurement axes of the accelerometer 6 are aligned closely enough with the reference frame of the user that the calibration step can be omitted.

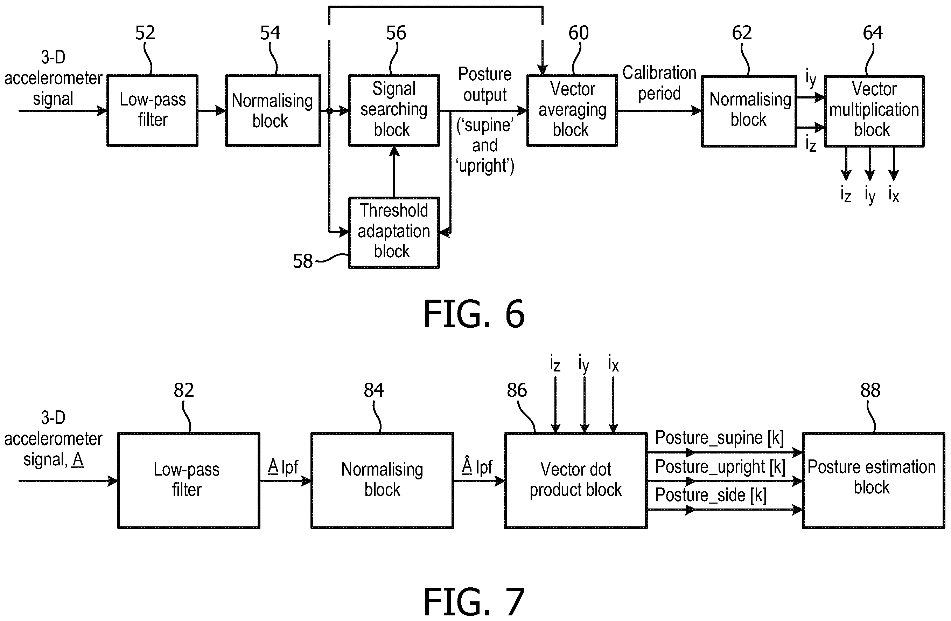

In an exemplary embodiment, the calibration step can be performed using an algorithm as illustrated in block form in Fig. 6. This algorithm is intended to improve the reliability of the detection of two or three of the main postures that occur while a user is in bed, during a bed exit and/or out of the bed. Those skilled in the art will appreciate that alternative techniques to that described below may be used to implement the step of calibrating the device.

The calibration procedure operates as follows. First, the three components of the 3D acceleration signal (i.e. x-, y- and z-axis signals) are smoothed by a low-pass filter in block 52. A filter of low computational complexity that can be used in this block 52 is a moving average (MA)-filter, which replaces each sample in each of the three measurement signals by the average of that sample and its neighbouring samples (i.e. the samples that are immediately preceding and following the current sample). A typical half-window size for the MA-filter is 1.6 seconds, although it will be appreciated that other window sizes can be used.

Subsequently, in normalising block 54, the low-pass filtered signals are normalised. In particular, each 3D sample (i.e. x, y and z value at a particular time instant) is considered as a 3-dimensional vector and is normalised to unit length. This normalisation is performed to enable the thresholding step described below. However, those skilled in the art will appreciate that the thresholding step described below can alternatively be implemented without having first normalised the signals.

In block 56, the normalised signal is searched for a first period of time (set of samples) in which the user is in a first posture and at least a second period of time in which the user is in a second (different) posture. For example, block 56 can search the normalised signal for a period in which the user is lying supine, and in particular, the normalised signal is searched for a contiguous time period during which the z-component exceeds a threshold. The contiguous time period could be as short as 1 second, but a typical initial minimum duration for an identified time period to be considered 'contiguous' is 15 seconds. The longer the minimum duration required to be identified, the better the reliability of the algorithm.

The normalised signal has a value in the range [1, -1], and the z-component should be the largest when the user is lying down. A typical initial value for the threshold (when the signal is normalised and when the threshold can be adapted as described below) is 0.6. If threshold adaption as described below is not used, a typical value for the threshold can be 0.8. If a contiguous time period is identified, this period is considered to be a time period when the user is lying supine. The algorithm further comprises a threshold adaptation block 58 that is configured to adapt the threshold if the search block 56 finds the end of a contiguous time span (i.e. if the previous sample in the search was above the threshold and the current sample is below the threshold) and the length of the current time span exceeds the length of the last used time span (the last used time span being the last contiguous time period in which the z-component exceeded the threshold). In one embodiment, block 58 is configured to set the threshold to the mean of the z-component (of the normalised signal) in the last used time span. In addition, the parameter 'length of last used time span' is updated to reflect the length of the contiguous time span that has just ended.

It is clear that the above algorithm can be refined in several ways. For example, the signal searching and threshold adaptation may stop (freeze) after, for example, 10 minutes of usage, or after having been updated a certain number of times (for example only once).

It will be appreciated that the signal searching block 56 can identify when the user is sitting or standing upright by searching for a contiguous period in which the y-component (preferably of the normalised signal) is above a threshold value. The initial threshold value can be as indicated above for the supine posture (i.e. 0.6), but the initial minimum duration for the contiguous period will be shorter, typically 2-5 seconds.

Likewise, the signal searching block 56 can identify when the user is lying on their side by searching for a contiguous period in which the magnitude of the x-component (preferably of the normalised signal) is above a threshold value. The threshold value will differ for determining whether the user is lying on their left or right sides.

To align the measurement axes of the accelerometer 6 to the reference frame of the user, the three-dimensional mean of the full three-dimensional normalised signal is computed over the each of the identified time periods, for two identified postures (for example an identified 'supine' posture and an identified 'upright' posture in Fig. 6). This is performed by vector averaging block 60 in Fig. 6. It will be appreciated that the three-dimensional mean (which is a vector) is calculated by averaging the value of the signal in an identified time period for each measurement axis (e.g. the x-component of the acceleration signal in the identified time period is averaged to give a value for the x-component of the three-dimensional mean).

These means are then normalised in normalising block 62. The normalised mean of the 'supine' time period yields the (now calibrated) 'off-body' ("z") direction $i_z$. The normalised mean of the 'upright' time period yields the (now calibrated) 'down-top' ("y") direction $i_y$.

Given these two calibrated directions, the 'lateral' ("x") direction is defined to be perpendicular to these two: $i_x$ = cross($i_y$, $i_z$), which is calculated by vector multiplication block 64. The lateral direction is also normalised (but that is the case if $i_y$ and $i_z$ are already normalised). It will be noted that $i_z$ and $i_y$ may not be orthogonal following the calibration.

In pseudo (MatLab) code, blocks 60 and 62 can be implemented using:

- for d = 1:3, $i_z$(d) = mean(accNormalized (kSup0:kSup1,d)); end
- accSz = norm($i_z$,2);
- $i_z$= $i_z$/accSz;

where accNormalized is the (3D) accelerometer signal of which each sample has been normalized, kSup0:kSup1 indicates the range of samples (the (last) time period that passed block 56), d is the dimension (x,y,z), $i_z$ is the resulting three dimensional mean (a vector of three components). The division by accSz normalizes this $i_z$. $i_y$ is calculated in the same way (but substituting $i_z$ by $i_y$ and kSup0:kSup1 by kUpr0:kUpr1 (for the samples from the found upright period). $i_x$ follows from the vector cross product $i_y$ x $i_z$ (block 64).

The resulting $i_x$, $i_y$, and $i_z$ are used in the subsequent processing to determine the change in height and estimate the posture(s) of the user. If this calibration step is omitted $i_x$= (1,0,0), $i_y$ (0,1,0), and $i_z$= (0,0,1) are used instead.

It will be appreciated by those skilled in the art that one or both of the normalisation operations described above can be omitted or modified, subject to suitable modifications to the operation of the vector averaging block 60 and vector multiplication block 64. Alternatively, or in addition, it will be appreciated that the vector averaging block 60 does not have to compute the mean of the three-dimensional (normalised) acceleration vectors, but could determine an alternative measure of the average of the three-dimensional acceleration vectors over the each of the identified time periods.

Posture estimation

In a preferred embodiment, step 109 of estimating a posture is performed using an algorithm as illustrated in block form in Fig. 7. The algorithm aims to determine the presence of the three main postures that occur during a bed exit in the accelerometer signals, namely lying supine, lying on their side and standing upright.

The first part of the algorithm corresponds to the calibration algorithm described above. In particular, the three components of the 3D acceleration signal (i.e. x-, y- and z-axis signals, denoted A) are smoothed by a low-pass filter in block 82 (the filtered signal being denoted $A_{lpf}$. A filter of low computational complexity that can be used in this block 82 is a moving average (MA)-filter. A typical half-window size for this MA-filter is 1.6 seconds, although it will be appreciated that other window sizes can be used. A normalising block 84 normalises the low-pass filtered signals. The normalised low-pass filtered signal is denoted $\hat{A}_{lpf}$. It will be appreciated that this algorithm may make use of the same low-pass filtering block and normalising block as shown in Fig. 6.

After normalising the low-pass filtered signals, a signal is calculated from the low-pass filtered signals and the (calibrated) axis vectors $i_x$, $i_y$ and $i_z$ for each of the postures: lying supine, lying on their side and standing upright.

Specifically, a vector dot product block 86 is provided that receives the normalised low-pass filtered signals $\hat{A}_{lpf}$ and the (calibrated) axis vectors and computes dot products for each sample k as follows:

$$\text{Posture\_supine}[k] = \text{dot}(i_z, \hat{A}_{lpf}[k]) \tag{1}$$

$$\text{Posture\_upright}[k] = \text{dot}(i_y, \hat{A}_{lpf}[k]) \tag{2}$$

$$\text{Posture\_side}[k] = \text{dot}(i_x, \hat{A}_{lpf}[k]) \tag{3}$$

It will be appreciated that the low-pass filtered signals $\hat{A}_{lpf}$ and the (calibrated) axis vectors have a magnitude of 1 (since both vectors have been normalised), so the vector dot product results in a value that is equal to the cosine of the angle between the two vectors. Therefore, vector dot product block 86 effectively determines a measure of the correspondence in direction of the normalised low-pass filtered signals $\hat{A}_{lpf}$ and the (calibrated) axis vectors. The greater the correspondence in direction (i.e. the closer to parallel the vectors are), the higher the value output for that posture by block 86.

Then a posture estimation block 88 determines the posture for each sample k. In particular, the posture at sample k corresponds to the posture signal with the largest 'presence'. For example, the absolute value or magnitude can be determined for each posture signal and the absolute values compared.

The current posture is determined to be supine at sample k if |Posture_supine[k]| is larger than |Posture_upright[k]| and |Posture_side[k]|. The current posture is determined to be upright at sample k if |Posture_upright[k]| is larger than |Posture_supine[k]| and |Posture_side[k]|. The current posture is determined to be lying on their side at sample k if |Posture_side[k]| is larger than |Posture_upright[k]| and |Posture_supine[k]|.

In case only two postures are considered by posture estimation block 88, Posture_supine[k] and Posture_side[k] can be combined into a single metric, Posture_lying[k], by vector dot product block 86, and then Posture_lying[k] can be compared to Posture_supine[k] by posture estimation block 88. The posture can be determined to be upright at sample k if |Posture_upright[k]| is larger than |Posture_lying[k]| and is determined to be lying if |Posture_upright[k]| is less than |Posture_lying[k]|.

It will be appreciated that the determined postures relate to the orientation of the upper body of the user to which the device 4 is attached. It is not necessary to determine the orientation of the legs (e.g., horizontal in bed) in order to determine whether the user is going to get out of bed.

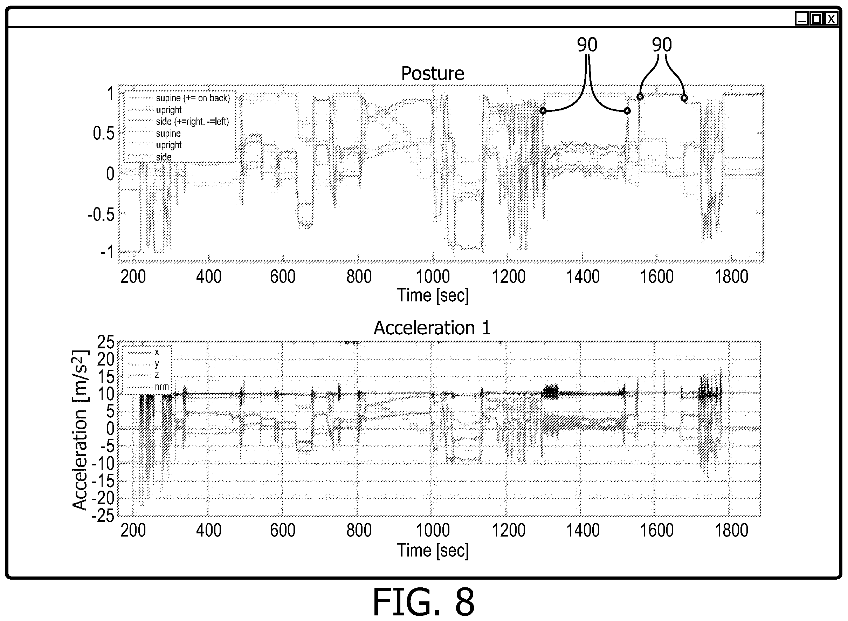

Fig. 8 shows an exemplary estimated posture signal graph (top graph) and signals from an accelerometer 6 used to derive the posture signal (bottom graph). The dashed lines in the posture graph depict the estimated posture signals (Posture_supine, Posture_upright and Posture_side) before calibration, where $i_x = (1,0,0)$, $i_y$ (0,1,0), and $i_z = (0,0,1)$ is used, and the solid lines depict the estimated posture signals (Posture_supine, Posture_upright and Posture_side) following calibration. Circles 90 indicate the selected regions for the calibration procedure, i.e. the samples between the kSup0:kSup1 and the kUpr0:kUpr1 ranges identified above.

At 800 seconds a change in the upright posture can be seen due to this calibration. This will have the effect that the output of the posture estimation algorithm described above at this time will be an upright posture rather than a supine posture. In fact, at 800 seconds the user was sitting upright and the head end of the bed was being lowered.

As with the calibration procedure described above, it will be appreciated by those skilled in the art that the normalisation operation described above (performed by block 84) can be omitted or modified, subject to suitable modifications to the operation of the vector dot product (vector multiplication) block 86.

Height estimation

As described above, the height of the device 4 can be estimated from the accelerometer signals as described in Appendix 1. Also as described above, the height change can also be calculated using the measurements from an air pressure sensor 30.

As described above, Fig. 3 is a graph illustrating the height estimated from accelerometer measurements and Fig. 4 is a graph showing an exemplary air pressure sensor signal obtained during a bed exit.

In this example, the device 4 is attached to the user's waist and is lying supine on the bed at time t = 135 seconds. At around 145 seconds, it can be seen that there is an increase in height, which corresponds to the user turning onto their side and rising leftwards in one movement so that they end up standing next to the bed. In Fig. 4, it can be seen that there is an apparent change in height at 138 seconds, but a comparison with the height change estimated from the accelerometer measurements shows this to be due to an environmental barometric fluctuation. The actual height rise is visible at 145 seconds.

Given the estimated height from the accelerometer and/or air pressure sensor measurements, a height difference is computed as the difference in estimated height between the current sample k and an earlier sample. Preferably the earlier sample is selected from the samples occurring around 10 seconds before the current sample.

As described above with reference to step 107 in Fig. 5, the estimated change in height is compared to a threshold, with an exemplary value for the threshold being 4 centimetres. The change in height will exceed this threshold value if a bed exit has occurred.

Temporal order

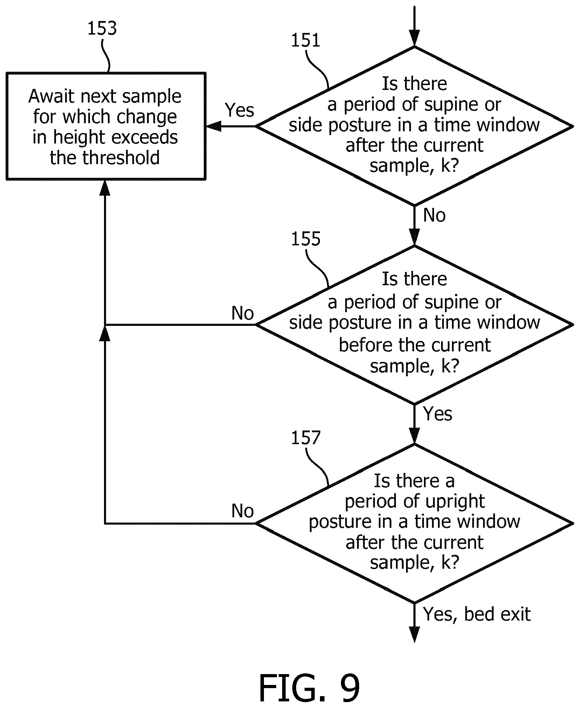

In step 111 of Fig. 5, it is determined whether the estimated change in height coincides with the postures expected to occur during a bed exit. An exemplary method of executing step 111 is shown in Fig. 9.

The method starts when step 107 in the flow chart of Fig. 5 indicates that the change in height for a sample k exceeds the threshold. In the first step, step 151, it is determined whether there is a supine/prone or a side posture within a time window after the current sample k. This time window can be, for example, the next 10 seconds after sample k, although it will be appreciated that windows of other lengths can be used. If there is a supine/prone or side posture in that window, regardless of the duration of that posture, then it is determined that the height change does not correspond to a bed exit and the method passes to step 153 to await the next sample for which the change in height exceeds the threshold.

If there is no supine/prone or side posture in the time window following the current sample k, then it is determined in step 155 whether there is a period of supine/prone (or a period of supine/prone or side) posture in a time window before the current sample k. This time window can be for example, the 20 seconds before the current sample k, although it will be appreciated that windows of other lengths can be used. The required period of supine or prone posture within the time window can be of the order of 2.5 seconds

To obtain a positive output of step 155, it may be necessary for the posture to be determined to be supine or prone continuously for the whole of the required period (i.e. for a complete period of 2.5 seconds within the 20 second time window). Alternatively, it may only be necessary for the posture to be supine or prone for at least a predetermined amount of the required period. The predetermined amount may be 80%, although it will be appreciated that other values may be used.

If there is no period of supine, prone or side posture in the time window before the current sample k that meets the requirements above, the algorithm moves to step 153 as described above.

If there is a period of supine, prone or side posture in the time window before the current sample k, then the method moves to step 157 in which it is determined whether there exists a period of upright posture in a time window after the current sample k. The time window can be, for example, 10 seconds after the current sample k, although it will be appreciated that windows of other lengths can be used. The required period of upright posture within the time window can be of the order of 2.5 seconds.

As above, it may be necessary for the posture to be upright continuously for the whole of the required period (i.e. for a complete period of 2.5 seconds within the 10 second time window). Alternatively, it may only be necessary for the posture to be upright for at least a predetermined amount of the required period. The predetermined amount may be 80%, although it will be appreciated that other values may be used.

It will be appreciated that in the posture estimation described above, the posture of the user will always be classified as one of supine/prone, side or upright, and therefore step 157 can be omitted since this result has already been determined by the negative response in step 151. However, this step can be used when an alternative posture estimation algorithm is employed (i.e. where more than three postures can be identified). If there is no period of upright posture in the time window after the current sample, the method returns to step 153.

If there is a period of upright posture in the time window after the current sample, a bed exit has been identified.

It has been found that this embodiment results in a reliable detection of bed exits while minimising the occurrence of false alarms. For example, it has been found that the change in height of 4 centimetres can be detected while the user is walking (since the upper body is moving up and down by this amount), but there will not be a period of prone/supine or side posture within a small time window around these height changes. It has also been found that the sequence of lying followed by upright also happens while staying in bed, but in this case there will not be a height change sufficient to exceed the threshold. The temporal order of lying supine/prone or on their side followed by an upright posture more-or-less centered around a height change has been found to be unique for a bed exit.

However, it has also been found that it is possible that a bed exit will not be determined for a person that is sitting upright for a while and then leaves their bed. In this case, step 155 will result in a negative output and the bed exit will not be detected.

One way of overcoming this problem is for the device 4 to be able to detect when the user has turned about 90 degrees around the vertical (i.e. detecting when the user has moved from a sitting position facing the end of the bed to a sitting position facing the side of the bed). It is possible to determine this solely from the accelerometer measurements, but the presence of a further sensor, such as a magnetometer 32 or gyroscope 34, is preferred.

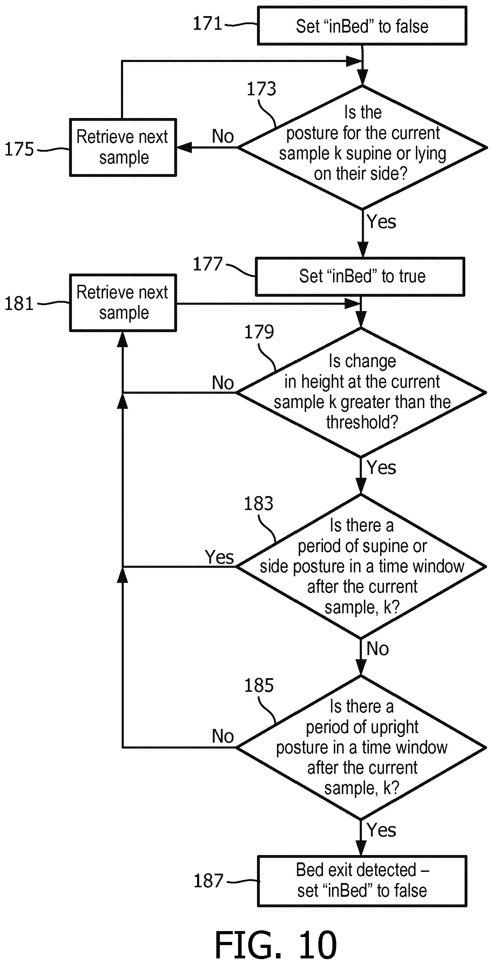

An alternative solution to the problem of the user sitting for a prolonged period of time before exiting the bed is as follows. A Boolean term "inBed" is introduced, and the principle is that this Boolean records whether a supine/prone or side lying posture has occurred without an increase in height occurring since then. This method is performed as shown in Fig. 10 and replaces steps 107 and 111 in Fig. 5.

At initiation of the method, in step 171, the Boolean "inBed" is set to false.

In step 173, it is determined whether the posture for the current sample k is supine or lying on their side (or just lying if a single measure replaces the supine and side signals). This step corresponds to the operation of the posture estimation block 88 in Fig. 7. If not, the method moves to step 175 in which the next sample is retrieved and step 173 repeated for that sample.

If the posture for the current sample is supine or lying on their side, then "inBed" is set to true (step 177).

In step 179, it is determined whether the change in height at the current sample k is greater than the threshold value (although it will be appreciated that this step can be omitted if it has already been performed in step 107 of Fig. 5).

If the change in height is not greater than the threshold value, then the method moves to step 181 in which the next sample is retrieved. Step 179 is then repeated for the next sample.

If the change in height is greater than the threshold value, then the method moves to step 183 in which it is determined if there is a period of supine or side posture in a time window after the current sample, k. The time window can be, for example, the period of 10 seconds after the current sample k, although it will be appreciated that windows of other lengths can be used.

If the user is in a supine or side posture in that time window, the method passes to step 175 (or step 185 since "inBed" will remain set to true).

If the user is not in a supine or side posture in that time window, then it is determined if there is a period of upright posture in a time window after the current sample k (step 185). The time window can be, for example, the period of 10 seconds after the current sample k, although it will be appreciated that windows of other lengths can be used.

If there is no period of upright posture in the time window after the current sample, the method passes to step 175 (or step 181 since "inBed" will remain set to true).

If there is a period of upright posture in the time window after the current sample, a bed exit has been identified and the Boolean "inBed" is now set to false (step 187).

It will be appreciated that in the posture estimation described above, the posture of the user will always be classified as one of supine/prone, side or upright, and therefore step 185 can be omitted since this result has already been determined by the negative response in step 183. However, this step can be used when an alternative posture estimation algorithm is employed (i.e. where more than three postures can be identified).

It will also be appreciated that step 187 in Fig. 10 can be split into two separate steps, with the output of step 185 leading to both a step in which "inBed" is set to false and then returns to step 175 and a step that triggers a bed exit alert with a care provider and/or feedback to the user.

It will also be appreciated that variations to the method presented in Fig. 10 are possible. For example, step 181 could be omitted and the inputs to step 181 provided directly to step 175. Then, step 173 could be preceded by a step in which it is checked whether "inBed" is true, and if so, the method passes to step 179. Otherwise, the method performs step 173 and then either step 175 or 177 depending on the outcome.

It will also be appreciated that it is possible to use a combination of the two algorithms presented above to determine the temporal order of the height change and postures, as well as other algorithms apparent to those skilled in the art. For example, while using the "inBed" parameter, the time period between the last "lying" posture and the height change could be used as a likelihood parameter to decide if a bed exit has occurred.

It will also be appreciated that the above algorithms operate on a per sample basis, which is relatively computationally intensive. Modifications are possible in which only a subset of the samples are subject to the algorithm, thereby reducing the computational burden.

There is therefore provided a bed exit monitoring system that can detect when the patient or user is about to get out of bed (in addition to detecting that the patient or user has already got out of bed), thereby providing an alarm to nursing staff as early as possible, whilst minimizing the occurrence of false alarms.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### Appendix - "Estimating a height or a change in height from measurements from an accelerometer

As described above, where the device 4 does not include any sensors in addition to the accelerometer 6, the height or change in height of the device 4 can be determined solely from the accelerometer measurements as described in European patent application no. 61/524813 (U.S. Patent Application). entitled "Estimating velocity in a horizontal or vertical direction from acceleration measurements", which was filed on 61/524813 (U.S. Patent Application). in the name of Koninklijke Philips Electronics N.V. The technique for estimating velocity and height or a change in height described in that patent application is set out below and shown in Figs. 11 to 21 of the accompanying drawings. It will be appreciated by those skilled in the art that although the technique is presented primarily in terms of measuring a height or change in height that may occur during a fall by the user, it is equally applicable to determining a change in height that occurs when a user gets out of bed.

Briefly, the method of determining an estimate of the velocity of a device in a vertical direction according to the illustrated embodiments of the invention comprises obtaining measurements of the acceleration acting in a vertical direction on the device 4 using the accelerometer 6, using a first filter to remove acceleration due to gravity from the obtained measurements to give an estimate of the acceleration acting in a vertical direction due to motion of the device 4, integrating the estimate of the acceleration acting in a vertical direction due to motion of the device to give an estimate of vertical velocity and using a second filter to remove offset and/or drift from the vertical velocity to give a filtered vertical velocity. One or both of the first filter and second filter is a non-linear filter.

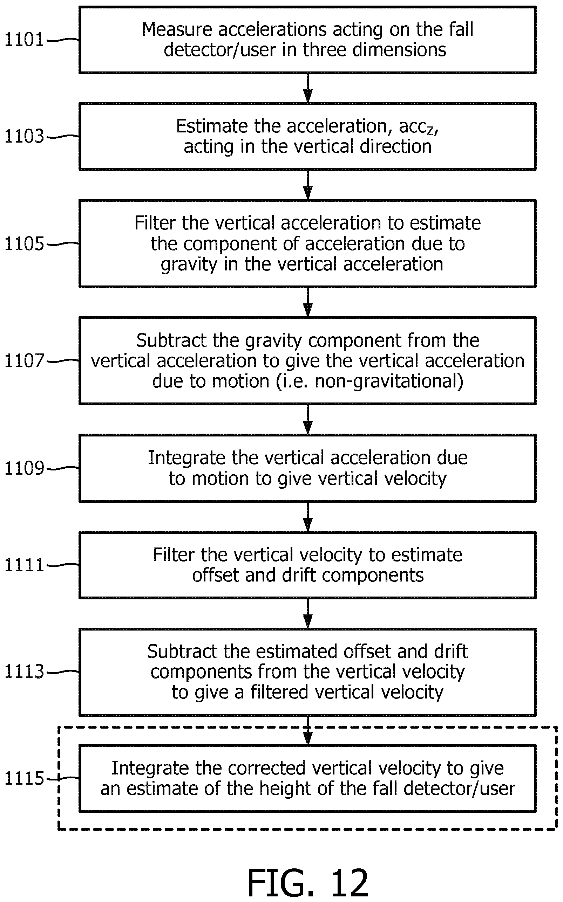

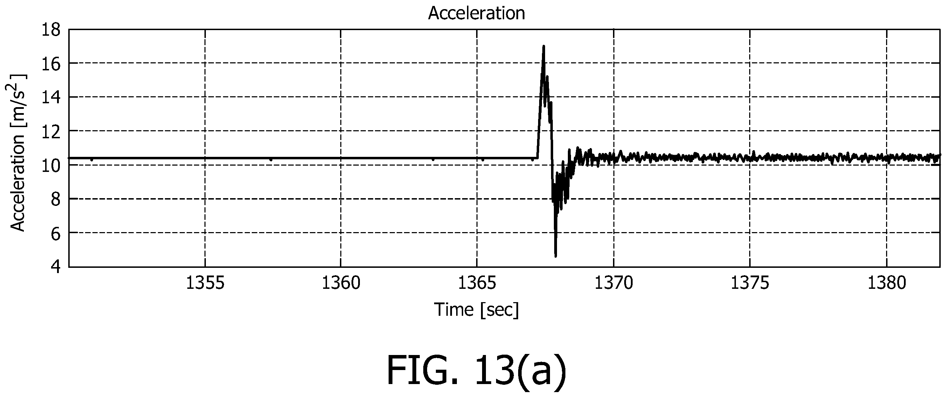

Fig. 11 shows a block diagram of the processing required to estimate the vertical velocity and then the change in height. It will be appreciated by those skilled in the art that these processing blocks can be implemented within the processor 8 of the device 4 or as separate electronic components. A flow chart illustrating a corresponding method of estimating the vertical velocity is shown in Fig. 12 (steps 1101-1113), along with the further step (step 1115) of estimating the height from the vertical velocity. Figs. 13(a)-(f) are graphs illustrating the signals at various stages of the processing shown in Fig. 11.

As an initial step (step 1101), a series of measurements of the acceleration acting on the accelerometer 6 (and therefore device 4) are collected. As indicated above, the accelerometer 6 measures acceleration in three dimensions and outputs a respective signal for each of the measurement axes.

The accelerometer measurements are provided to a first processing block 1022 that processes the measurements to identify the component of acceleration acting in the vertical direction. This processing is represented in Fig. 12 by step 1103, and can be performed in a number of different ways.

For an accurate estimation of the vertical acceleration to made, it is desirable to obtain an accurate estimation of the orientation of the accelerometer 6 (and therefore device 4) so that a coordinate transformation (rotation) can be applied to the accelerometer measurements.

This orientation estimation can be obtained when the device 4 comprises a further sensor, such as a gyroscope and/or magnetometer, and the output from these sensors, possibly together with that from the accelerometer 6, is used to determine the coordinate transformation (rotation) to be applied to the accelerometer measurements. After coordinate transformation, the vertical component of acceleration can easily be identified.

Alternatively, acceleration due to gravity (which by definition acts in the vertical direction) can be estimated as the low-pass component of the accelerometer measurements (making sure that the magnitude of the low-pass component is constant), and the direction that this component acts can be used to determine the (vertical) orientation of the accelerometer 6. The acceleration in the direction of the low-pass filtered acceleration will correspond to the acceleration in the vertical direction. As a yet further alternative, the output of a process that is similar to that performed by processing block 1024 when using a non-linear filter described below can be used to obtain an estimate of gravity and hence its direction (it is similar in the sense that it now operates on each of the components of the three-dimensional accelerometer signal).

A simpler way to estimate the vertical component of acceleration is to compute the norm of the 3D acceleration measurements. The signals output for each of the three measurement axes include gravity, which points in the vertical direction, and can be assumed to have a relatively large magnitude relative to accelerations due to motion. The accelerations due to motion and due to gravity combine as a vector sum. When computing the norm of this vector sum the contribution of the horizontal components are relatively small, since they are orthogonal to the gravity component and small in magnitude compared to this gravity component. Accelerations in the vertical direction will appear in the norm with an unaltered magnitude, provided that, when they are in a downward direction, they do not exceed gravity (otherwise the norm will turn the net negative component into a positive one, where an upward acceleration, such as by gravity alone, is defined positive). So, the norm is a cost-effective estimator of the vertical acceleration (including gravity). However, as suggested above, significant horizontal accelerations and large downward accelerations (i.e. exceeding gravity) will introduce distortions in the estimated vertical acceleration.

If the device 4 is implemented as, for example, a pendant to be worn around a user's neck, the device 4 will typically be in one particular orientation, and knowledge of this orientation can be used to identify the vertical component of acceleration from the accelerometer measurements. However, it will be appreciated that this approach is potentially subject to large errors if the device 4 is not worn properly or if its orientation changes during normal use or during a fall.

Another approach is described in WO 2010/035191 which describes a technique for estimating the vertical component of acceleration from a 3D accelerometer signal having an arbitrary orientation. According to that technique, the vertical component of acceleration is estimated by (i) examining the signals from the accelerometer to identify the axis of the accelerometer having the highest component of acceleration, (ii) determining the orientation of the accelerometer by determining the angle between the acceleration acting on the accelerometer (this acceleration being assumed to be generally due to gravity) and the axis with the highest component of acceleration and (iii) using the estimated orientation of the accelerometer to determine the acceleration in the vertical direction from the measurements of acceleration.

The vertical component of acceleration output by the first processing block 1022 is denoted $acc_z$ in Fig. 11, and an exemplary vertical component signal is shown in Fig. 13(a). This signal covers a period of time in which an increase in height occurs (around time 1368 seconds); where the device has been lifted from a stationary position on a desk and held in the hand, which induces some vibration. The vertical component of acceleration is provided to a second processing block 1024 and to an addition/subtraction block 1026.

Processing block 1024 estimates the acceleration due to gravity in the vertical component of acceleration (corresponding to step 1105 in Fig. 12) using a first filter.

In a simple embodiment, processing block 1024 uses a constant value for gravity. This value may be 9.81 ms$^{-2}$, but it may be a different value, depending on the particular characteristics or calibration of the accelerometer 6. For example, it is not uncommon for an accelerometer 6 to output values for acceleration that are out from the actual values by 0.2 ms$^{-2}$ or more, and this can be factored in to the constant value used. This can be seen in Fig. 13(a) where the constant value is greater than 10 ms$^{-2}$. In this simple embodiment, processing block 1024 can apply an estimator to the vertical component of acceleration that outputs a constant value for gravity (e.g. 9.81 ms$^{-2}$). As known, apart from the constant output, the estimator can be understood to be a filter.

In an alternative embodiment, processing block 1024 can apply a linear filter to the vertical component of acceleration to provide the estimate for gravity. The linear filter can be a low-pass filter with an appropriate cut-off frequency. For example, the filter can be a moving average filter.

As known, a linear filter is characterized by its impulse response curve. A pulse at its input results in a signal that is spread in time. Therefore, a sudden change in acceleration, which happens during a fall, can be seen as a pulse superimposed on the continuously sensed gravity signal. Consequently, when estimating a gravitational component using a linear filter, there will be an over and under estimation due to the impulse spread. The severity of the over or under estimation depends on the bandwidth of the filter (or length of the impulse response). These over and under estimations will be treated in the subsequent integration step as part of the vertical acceleration due to motion and hence will lead to an erroneous velocity estimate, and thus position estimate too.

Furthermore, the component of gravity along each of the measurement axes of the accelerometer 6 changes when the orientation of the accelerometer 6 changes. This change appears as a transient error in the estimated gravity. The transient error is similar to the impulse response and has the same spread (more precisely, it is a step response). This spread also results in an error in the velocity and position estimate after the integration steps. This type of error is not present in the embodiment where the norm is used, assuming no calibration error, since the norm is insensitive of orientation.

Where the vertical component of acceleration is estimated (as in the process described above), this orientation change is taken into account, and the effect is reduced to the level of errors in the orientation estimated by block 1022 (and in step 1103). In the event that the measurements from the accelerometer 6 include an offset (e.g. the 0.2 ms$^{-2}$ mentioned above), the change in orientation will cause a change in magnitude of the sensed gravity component due to that offset. This is another effect that can cause transient errors, which again may appear as errors in the position estimate if not properly filtered.

Therefore, in view of these problems, processing block 1024 applies a non-linear filter to the vertical component of acceleration to provide an estimate for gravity. This is because a non-linear filter is much more capable of "ignoring" the sudden change in acceleration that occurs during a fall, or of following the transient that occurs upon a change in orientation.

In one preferred implementation, the non-linear filter can be a median filter. As known, a median filter processes each sample in the input signal in turn, replacing each sample with the median of a number of neighbouring samples. The number of samples considered at each stage is determined by the window size of the filter. A typical half window size can be 1.6 seconds (so the window encompasses 1.6 seconds worth of samples before the current sample and 1.6 seconds worth of samples after the current sample).

A median filter is known for suppressing pepper-and-salt noise in video images, i.e. suppressing (black-and-white) signal spikes of short duration. The accelerations experienced during a fall can be considered as a spike in the acceleration signal of (relatively) short duration, and therefore removing this spike using a median filter will therefore produce a much better estimate of gravity, and does not suffer from the response spread problems associated with linear filters.

In another particularly preferred implementation, the non-linear filter can be a recursive median filter. This type of filter has the property that it tends to stick to its previous estimates (of gravity). In this way, a fluctuation in the acceleration does not immediately appear as crosstalk in the estimate of the gravity component, while a change, e.g. a step due to an orientation change and poor calibration of the accelerometer 6, is still followed.

As known, a recursive median filter is similar to a median filter, except that in computing the median for a particular sample it uses the already-computed median values in the sample window, rather than the original sample values in the signal.

The recursive median filter can be a forward or backward recursive median filter, which determines the direction in which the vertical acceleration signal is filtered.

The forward recursive median filter will try to keep the past values (i.e. keep them constant), while the backwards recursive median will try to keep the future values. Depending on the nature of the signal, the output of each of these types of recursive median filter can be different. For example, where the signal before a pulse has a lower value than after the pulse, a forward recursive median filter will tend to use the lower value, while the backwards recursive median filter will tend to use the higher value, and there will be a difference between the two outputs. Thus, it is possible to apply both filters separately to the vertical acceleration signal and average the results to obtain the gravity component. Alternatively linear interpolation between the points where the two filter outputs diverge can be used. Those skilled in the art will appreciate that it is possible to use other forms of combining the two results.

In an alternative implementation, the non-linear filter is a weighted median filter, in which a respective weight is applied to each sample in the filter window.

In yet another alternative implementation, the non-linear filter is a mode filter. This filter takes the sample value to which most of the sample values in the current window are closest.

In another implementation, hybrid versions are used to filter the vertical acceleration. This filter estimates gravity and a decision process is run to decide what value to use. This decision can be to use a combination of estimates by different filters or to freeze the estimated gravity when the level of motion exceeds a threshold, for example.

In another implementation (although more complex than the use of a normal median filter or recursive median filter), a modified median filter, referred to herein as a subMedian filter, is used to filter the vertical acceleration. The subMedian filter is described in more detail below.

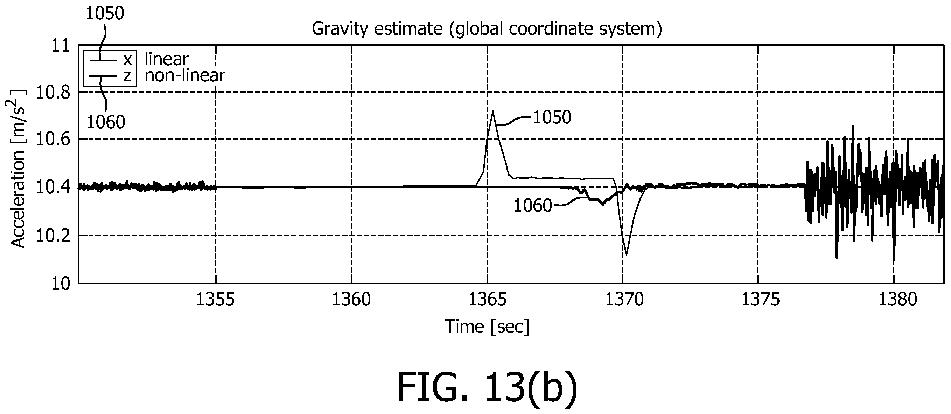

Fig. 13(b) shows the estimate of the acceleration due to gravity output by processing block 1024 when a linear low-pass (moving average) filter is applied to the vertical acceleration signal (represented by line 1050) and when a non-linear median filter is applied to the vertical acceleration signal (represented by line 1060).

Although not illustrated in Fig. 11, it is possible to apply a further filter to the estimate of the gravitational acceleration to smooth the signal.

Where the device 4 is also configured to process the accelerometer measurements to determine if the user is getting up following a detected fall, it is possible to time-shift the estimated acceleration due to gravity by one or two seconds. When the user is lying on the floor, the signal for the gravitational component is relatively smooth (i.e. constant). Therefore, by time-shifting the gravitational estimate, a constant value can be used during the period where the user might be getting up.

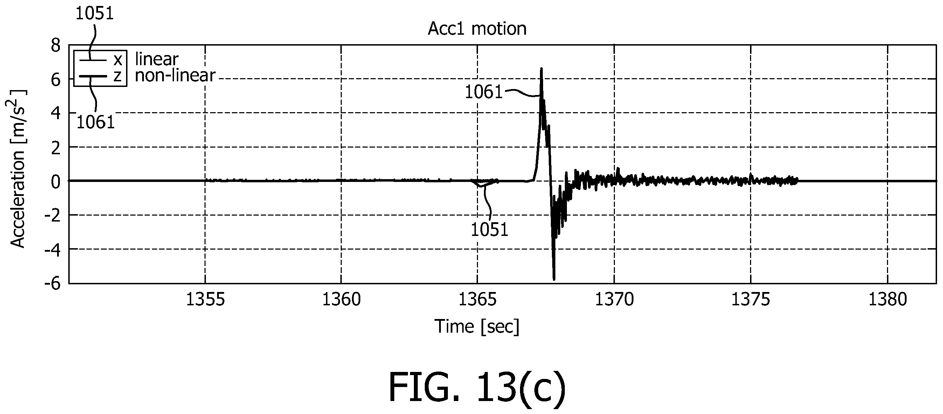

The estimate of the acceleration due to gravity output by processing block 1024 is provided to the addition/subtraction block 1026 where it is subtracted from the vertical component of acceleration output by the first processing block 1022 to leave the acceleration in the vertical direction due to the motion of the device 4 (step 1107). The estimated vertical acceleration due to motion output by block 1026 (after subtracting the gravity estimate obtained using the non-linear median filter - labelled 1061, and after subtracting the gravity estimate obtained using a moving average filter - labelled 1051) is shown in Fig. 13(c).

It will be appreciated that the output of processing block 1024 may be delayed with respect to the estimate of vertical acceleration provided directly to the addition/subtraction block 1026 due to the time required for the processing by processing block 1024. Therefore, the inputs to block 1026 can be synchronised (for example by introducing a delay into the vertical acceleration estimate, $acc_z$).

It will also be appreciated that the non-linear median filter 1024 that is applied to the vertical acceleration signal and the subsequent addition/subtraction block 1026 can be replaced by a single filter, referred to herein as a 'complementary' median filter, which acts in the opposite way to the median filter, i.e. it passes the parts of the signal blocked by the median filter and blocks the parts of the signal passed by the median filter. Thus, the 'complementary' median filter passes the pulses of short duration representing the vertical acceleration due to motion of the device 4 and removes the gravitational acceleration in the vertical acceleration signal. Referring to Fig. 12, this complementary filter would correspond to a combination of steps 1105 and 1107.

The signal representing the vertical acceleration due to the motion of the device 4 is then integrated with respect to time by processing block 1028 to give an estimate of the velocity in the vertical direction (step 1109). The initial velocity value $v(t_0)$ input to the integration block 1028 is unknown, but is typically assumed to be zero. In any case, the next filtering stage (described further below) removes offset and drift in the vertical velocity signal, and therefore the initial velocity component (if non-zero) will be substantially removed.

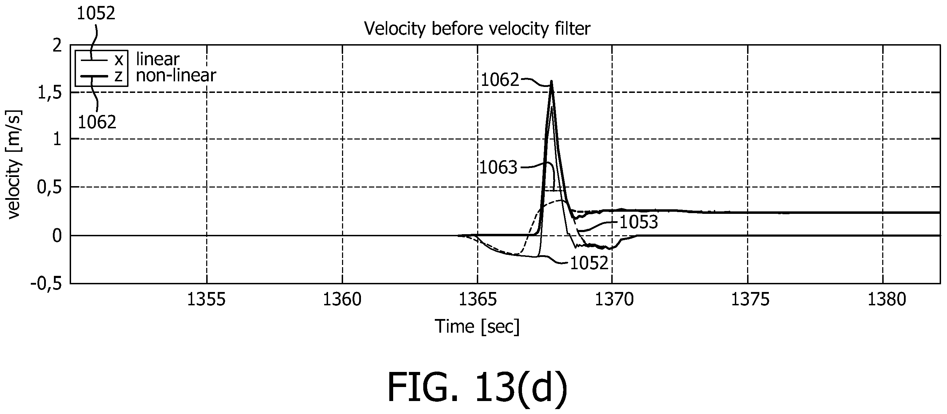

It has been found that the gravity-free acceleration signal (shown in Fig. 13(c)), is not a perfect representation of the accelerations due to the physical movement of the device 4. The signal is distorted, effectively causing an additional velocity component in the output of the integration block 1028. It has been assumed that the distortion is caused by the orientation estimation process performed by processing block 1022. The distortion is not constant, but relates to the movement signal, and therefore cannot be filtered as part of the gravity estimation by processing block 1024. However, after the integration by block 1028, the distortion primarily causes a monotonous component. This can be seen, for example, in the line labelled 1062 in Fig. 13(d) where the integration has left an offset of about 0.25 ms$^{-1}$ in the velocity. If a linear filter is used at the gravity estimation stage, the errors in the estimated gravity due to the filter response spread cause significant velocity components (shown in the line labelled 1052 in Fig. 13(d)).

Therefore, the signal representing the vertical velocity is provided to a fourth processing block 1030 which applies a filter to the vertical velocity signal to estimate the offset and any drift components present in that signal (step 1111). The result of this filtering is a signal representing the fluctuations of the monotonous (i.e. offset and drift) component.

Traditional linear filters to obtain a DC (constant) or slowly changing (offset and drift) component include low-pass filter and moving average filters (which also exhibits low pass behaviour). However, these filters affect adjacent samples through the time response corresponding to the filter. So, while an offset may be removed, a compensating "ghost offset" will appear before and after the corrected stretch of samples. These "ghost offsets" can significantly obscure the result of integrating the corrected stretch of samples to obtain a change in height.

Therefore, this problem is overcome by the processing block 1030 preferably applying a non-linear filter to the vertical velocity signal to remove the offset and drift present in the signal (step 1111).

In a preferred implementation, the processing block 1030 applies a median filter to the vertical velocity signal. As described above, the median filter effectively blocks pulses and oscillations in a signal while passing constant and edges (i.e. the offset and drift). A typical half window size for this filter can be 0.8 seconds (so the window encompasses 0.8 seconds worth of samples before the current sample and 0.8 seconds worth of samples after the current sample). In alternative preferred implementation, the processing block 1030 can apply a weighted median filter or a mode filter to the vertical velocity signal.

In a particularly preferred implementation, the processing block 1030 applies a median filter referred to herein as an 'adaptive median' filter. The adaptive median filter will be described in more detail below.

It will be appreciated that where the filter applied by processing block 1024 is a non-linear filter, the fourth processing block 1030 can apply a linear filter to the vertical velocity signal to estimate the offset and drift, depending on the application that uses the resulting velocity estimate. As can be seen in Fig. 13(d), the application of the non-linear filter in processing block 1024 has limited the spread in the obtained velocity. The application of a linear filter to remove the offset and drift will cause some spread, but, in the context of that application, of an acceptably limited extent.

The signal representing the offset and drift in the vertical velocity signal that is obtained using a non-linear median filter and that is output of the processing block 1030 is shown as dotted line 1063 in Fig. 13(d). A signal representing offset and drift in the vertical velocity signal that is obtained using a linear moving average filter and that includes a 'ghost offset' as described above, is shown as dotted line 1053 in Fig. 13(d).

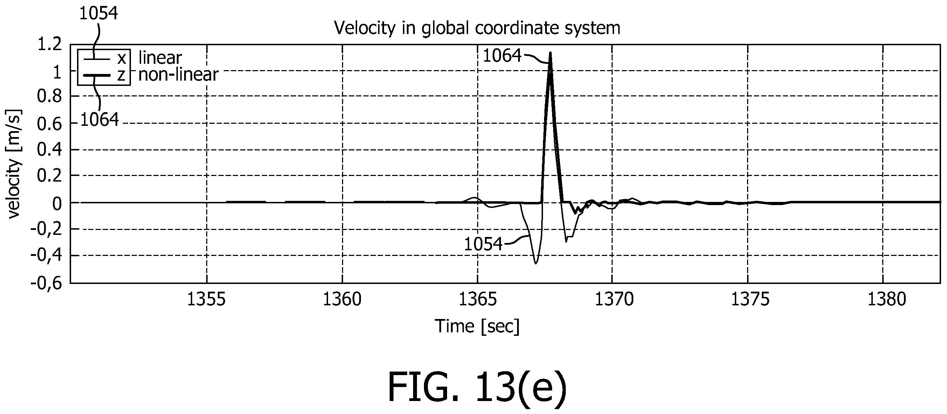

This signal is input to an addition/subtraction block 1032 along with the vertical velocity signal from integration block 1028, where it is subtracted from the vertical velocity signal to give an offset and drift free vertical velocity signal (step 1113). This signal is shown by line 1064 in Fig. 13(e). Thus, the non-linear filter(s) applied during the earlier processing stages result in a more accurate estimate of the actual vertical velocity of the device 4. The equivalent vertical velocity obtained through the application of a linear moving average filter to the estimate of vertical velocity is shown by line 1054 in Fig. 13(e), and it can be seen that part of the spread in the velocity has been removed, but a significant reversed component remains near the peak.

As with addition/subtraction block 1026, the inputs to addition/subtraction block 1032 may need to be synchronised to compensate for the delay introduced by processing block 1030.

It will be appreciated that the non-linear median filter 1030 that is applied to the vertical velocity signal and the subsequent addition/subtraction block 1032 can be replaced by single filter, referred to herein as a 'complementary' median filter, which acts in the opposite way to the median filter, i.e. it passes the parts of the signal blocked by the median filter and blocks the parts of the signal passed by the median filter. Thus, the 'complementary' median filter passes the pulses of short duration representing the actual velocity of the device 4 and removes the offset and drift present in the vertical velocity signal.

The offset and drift free vertical velocity signal is then integrated with respect to time by processing block 1034 to give the height or change in height of the device 4 (step 1115). The initial position value $p(t_0)$ input to the integration block 1034 will typically be unknown, but where the result of the integration is used to determine a change in the height, knowledge of the initial position is unnecessary. If it is desired to calculate the actual height, some calibration or initiation will be required in order to set $p(t_0)$.

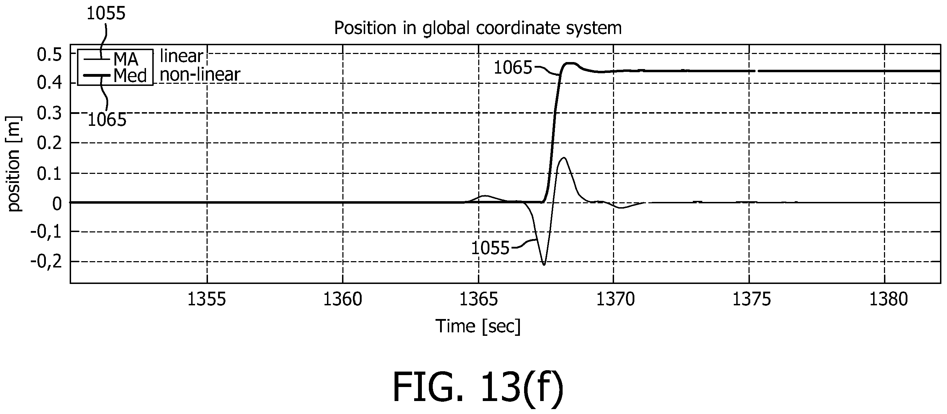

As suggested above, the device 4 is processing the accelerometer measurements in order to determine when there has been a change in height of the device 4 of a magnitude that corresponds to a fall by the user of the device 4. In addition, it will be appreciated that a change in height observed in the obtained height estimates that is indicative of a fall should be 'sustained', indicating that the device 4 is at a different height after the event than before. In other words, there should be a change in height from a first height before the fall to a second height (that is less than the first height) after the fall. Line 1065 in Fig. 13(f) shows the position estimates obtained when non-linear filters are used during the processing, and indicates that a 'sustained' height change has occurred (although it will be noted that this figure indicates an increase in height rather than a fall). Line 1055 in Fig. 13(f) shows the position estimates obtained when linear filters are used during the processing, and indicates that the erroneous velocity components that resulted from the filter response spreads compensate the rise completely, leaving effectively no (sustained) height change.

The output of integration block 1034 provides the estimate of height. A change in height, as used to detect a fall or a rise (standing-up), results from computing the difference between the estimated heights at two time instants, for example at the current time instant and at a couple of (e.g. 2) seconds ago. There are multiple ways in which the change in height can be used in the decision logic for detecting a fall. For example, it can be determined whether the computed change in height exceeds a (downwards) threshold. A more sophisticated example would be to use the size of the change itself in a probability metric.

The change in height can be computed directly from the velocity estimates output by addition/subtraction block 1032, bypassing integrator 1034, by using a 'moving integrator' or summer that integrates (sums) between the time instants between which the height change is to be computed. The moving summer is similar to a moving average filter, except that the division (averaging) by the moving average filter's window length is omitted. So, basically, the velocity samples in the window are summed and returned as the output, where the window is the region between the two time instants. The preferred approach (integrate first and compute differences or compute moving sum) is down to consideration of computational load. The choice can be influenced by other factors, such as, for example, the number of window sizes over which the height difference is to be computed. The implementation of the moving summer can be optimized using similar techniques as known for MA filter implementations.

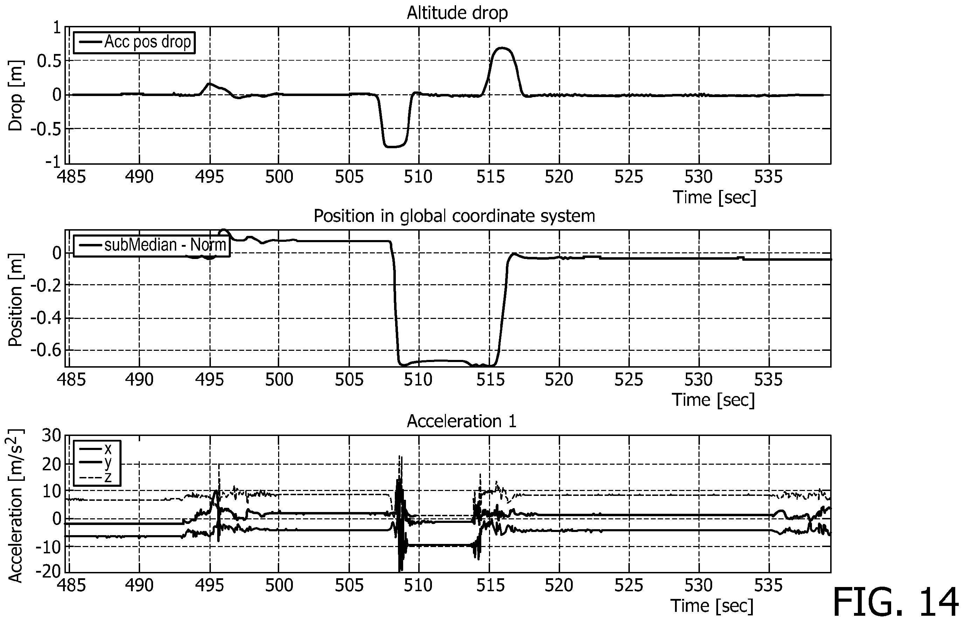

Fig. 14 shows the results of the processing according to the invention that is performed using a subMedian filter as the first filter 1024 and a median filter as the second filter 1030 on accelerometer measurements obtained during a fall. Thus, the graphs show the altitude drop (i.e. the change in height) and height (relative to an initial height of approximately zero) derived from the accelerometer measurements shown in the bottom graph. The height difference in the top graph is computed as the difference between the current height estimate and the height estimate a predetermined time period ago (in this example, the time period is 2 seconds). So, a drop in height results at the onset of the fall and remains visible for 2 seconds in the graph until the 'predetermined time period ago' passes the onset (i.e. 2 seconds later in this example).

Thus, in Fig. 14, there is a change in height indicative of a fall that occurs at around time t = 509 (see top graph) and this change in height results in a sustained difference in the height of the device 4 (e.g. see the middle graph - the height of the device 4 observed in a time window before the change in height is higher than the height of the device 4 observed in a time window after the change in height.

As described above with reference to Figs. 11 and 12, the acceleration acting in a vertical direction is estimated from the three-dimensional accelerometer measurements (for example by taking the norm of the accelerometer measurements) and then the filter is applied to the one-dimensional vertical acceleration estimate in order to estimate the acceleration due to gravity. However, in an alternative implementation, the first filter can be applied to the signals from each of the measurement axes of the accelerometer 6 in order to estimate the acceleration due to gravity in three dimensions, prior to the estimation of the vertical component of acceleration. This three-dimensional gravity estimate can then be used to estimate the vertical component of acceleration in the three-dimensional accelerometer measurements, whereafter the gravity estimate (or another gravity estimate obtained using a further filter - the same or a different type to the first filter) is subtracted from the estimated vertical component to give the estimated vertical acceleration due to the motion of the device. In addition, using this approach the horizontal accelerations (due to motion) can be estimated and be processed in a similar manner to estimate horizontal velocity and displacement.

subMedian Filter

As described above, in a preferred embodiment of the invention, a filter, termed herein as a 'subMedian' filter, is applied to the estimate of the vertical acceleration output by processing block 1022 in order to estimate the gravity component in that signal. Alternatively, the subMedian filter can be used to estimate the three-dimensional acceleration due to gravity in the three-dimensional signals received from the accelerometer 6.

Also as indicated above, the gravitational component in the acceleration signal is more or less constant (i.e. DC), it changes direction in the accelerometer's frame of reference when the accelerometer 6 and device 4 are rotated, and the norm of the three-dimensional accelerometer measurements change when the accelerometer 6 is not perfectly calibrated.

Therefore, the filter applied to the accelerometer measurements (or the estimate of vertical acceleration) is required to output the DC component in the signal and to follow changes (for example due to rotations) instantaneously (i.e. no spread should be introduced as a result of applying the filter).

A low-pass filter is able to return the DC component, but introduces spread and doesn't quickly follow changes when the orientation of the accelerometer 6 changes. A median filter, although generally providing an acceptable output, can drift when the input signal is more complex (for example, a gentle but continuous movement of the accelerometer 6 up and down over a short height difference, followed by a large height change and a continuation of the short up and down movement, may reveal a drift in the output representing the estimated gravity component).

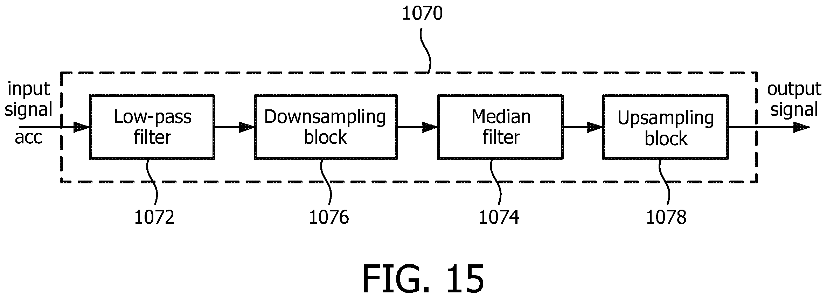

Therefore, a subMedian filter has been designed that combines a low-pass filter and a non-linear filter. Preferably this filter is a median filter or a median-based variant. An exemplary subMedian filter 1070 is shown in Fig. 15.

Thus, a signal is input to a low-pass filter 1072, and the low-pass filtered signal is input to a median filter 1074. The low-pass filter 1072 can have a larger bandwidth than when used alone, which allows the removal of some of the high frequency components in the input signal, but still allows quick movements to be followed.

The median filter 1074 can alternatively be a median variant, such as a recursive median filter or a weighted median filter.

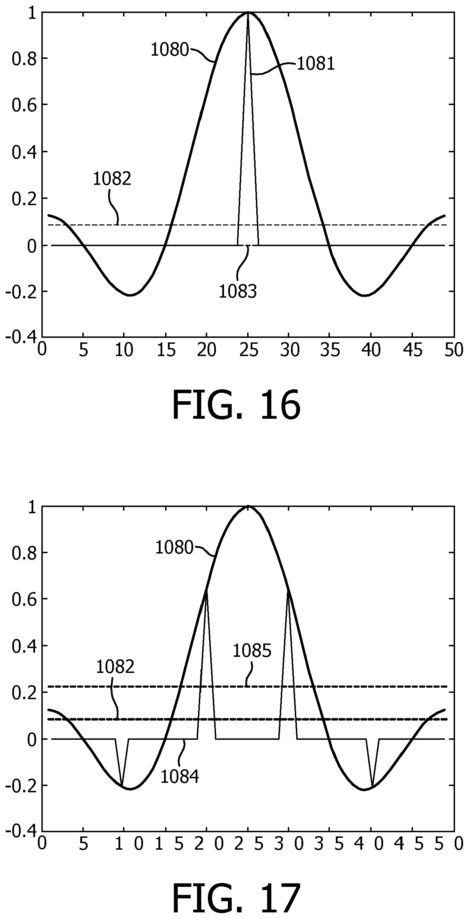

Since the input signal is low-pass filtered, according to the Nyquist-Shannon sampling theorem the low-pass filtered signal can be downsampled to the Nyquist rate corresponding to the bandwidth of the low-pass filter without aliasing effects. Downsampling to the Nyquist rate allows the operation of the median filter 1074 to be more effective in two ways. On the one hand it provides a computational gain, since fewer samples are in the filter's window. On the other hand, it is realized that the filter should be more effective in removing the unwanted components (spikes). This will be further explained below, referring to Fig. 16.

Thus, the subMedian filter 1070 further comprises a downsampling block 1076 between the low-pass filter 1072 and median filter 1074 that downsamples the low-pass filtered signal to the Nyquist rate (i.e. twice the cut-off frequency of the low-pass filter 1072). A parameter, termed the 'sub-sampling ratio' or 'subRatio' herein, determines the amount by which the sample rate of the low-pass filtered signal is reduced. A typical value for the subRatio is 20. The cut-off frequency for the low-pass filter 1072 is typically set less than the bandwidth of the signal at the sub-sampled rate (the bandwidth being half the sampling rate). The bandwidth of the signal at the sub-sampled rate can, for example, be multiplied by 0.8 to set the cut-off frequency for the low-pass filter 1072; the value of 0.8 being chosen to prevent aliasing effects upon downsampling. Thus, for a signal having a sampling rate of 50 Hz, the cut-off frequency for the low-pass filter 1072 will typically be 1 Hz, being 0.8 times half the sampling rate after downsampling (which is 1.25 Hz).

An upsampling block 1078 is provided to upsample the output of the median filter 1074 back to the sampling rate of the signal input to the subMedian filter 1070. As known in the art, the upsampling is followed by an interpolation filter, not shown in the figure, which is typically a low-pass filter of the same bandwidth as that one used in block 1072. The filters should be scaled to keep signal strength at identical level. Upsampling can be performed in several ways, as is known in the art. For example, one way is to insert additional samples of zero value. Another example is to use sample & hold, i.e. to insert samples of the same value as the last available sample.

Applying the median filter 1074 to a downsampled low-pass filtered signal makes the median filter 1074 more effective in removing a spike in the signal (i.e. the accelerations due to motion of an accelerometer 6).

Fig. 16 is a graph illustrating the basic principle of operation of the subMedian filter 1070. Line 1080 in Fig. 16 is a sinc signal corresponding to the bandwidth of the low-pass filter 1072. Line 1081 is the same sinc signal but sampled at the Nyquist rate, i.e. at the output of block 1076. It can be seen that the subsampling is precisely at the zero crossings of the sinc signal 1081 and therefore a pulse results in the signal.

Dashed lines 1082 and 1083 show the median-filtered value of signals 1080 and 1081 respectively. Thus, it can be seen that applying the median filter 1074 to the subsampled low-pass filtered signal results in a better outcome (the true DC value).

However, it will be appreciated that when sub- or down-sampling the low-pass filtered signal, the choice of the first sample is arbitrary. Fig. 16 shows the ideal case where the subsampling is precisely at the zero crossings of the sinc signal 1080. However, Fig. 17 illustrates the scenario where the sub-sampling results in samples halfway between the zero-crossings of the sinc signal (which is the worst case). Line 1084 corresponds to the subsampled signal and dashed line 1085 shows the median filtered signal.

Therefore the subMedian filter 1070 can be adapted for this phase effect in the subsampled low-pass filtered signal. In particular, the downsampling block 1076 can be configured to subsample at all phases, with the median filter 1074 being applied to each subsampled signal. The final output of the median filter 1074 can be a combination of the outcomes of the median filtering on each of the subsampled signals.

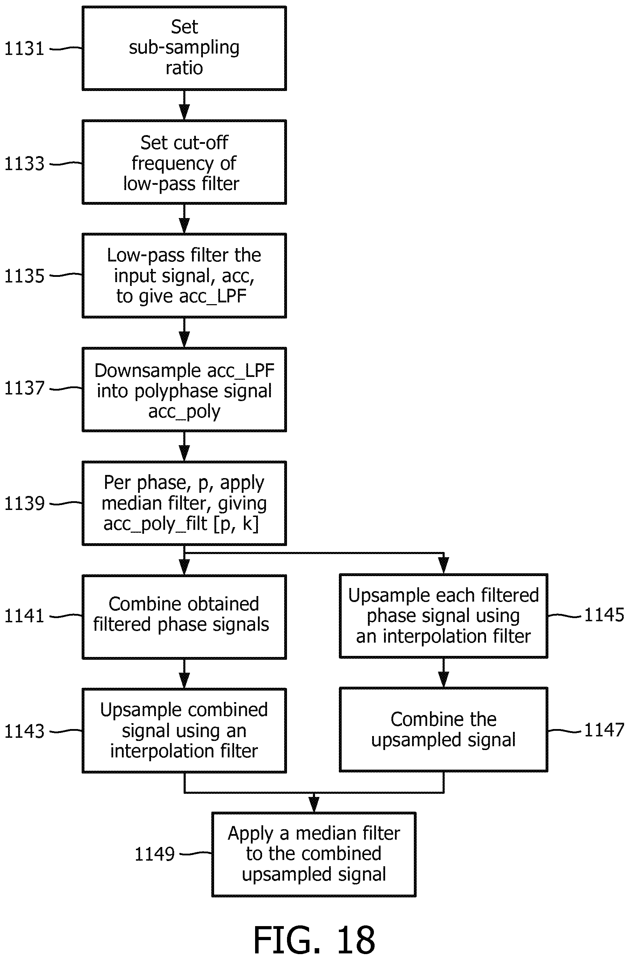

Fig. 18 is a flow chart illustrating the operation of an exemplary subMedian filter 70. In step 1131, the sub-sampling ratio, subRatio, is set, which can typically be 20 when the accelerometer signal is sampled at 50 Hz.

In step 1133, the cut-off frequency of the low-pass filter 1072 is set at 0.8 of Fs/2/subRatio (where Fs/2 is the full bandwidth of the signal, labelled 'acc' at the input of low-pass filter 1072).

In step 1135, the low-pass filter 1072 is applied to the input accelerometer signal, acc, to give acc_LPF.

In step 1137, the low-pass filtered accelerometer signal, acc_LPF, is downsampled into polyphase signal acc_poly according to the sub-sampling ratio. The number of ways acc_LPF can be subsampled is equal to the sub-sampling ratio. Hence, for each phase p, ranging from 0 to subRatio -1, the samples acc_poly at index k result as

$$\text{acc\_poly}[p,k] = \text{acc\_LPF}[p + \text{subRatio}*k] \qquad (A1)$$

In step 1139, the median filter 1074 is applied to the downsampled signal for each phase, p. This gives a set of signals denoted acc_poly_filt[p,k]. In this example, the half window size of the median filter 1074 is 1.6 seconds, although it will be appreciated that other values can be used.

Alternatively, as indicated above, other forms of median filters can be used, such as recursive median filters, or it is possible to iterate the (median) filter process (i.e. by repeating the median filtering several times on the outcome of the previous round).

Then, in step 1141, each of the obtained filtered phase signals is combined into a single signal. The combination of the signals can be effected by taking the mean or median of each sample k over the subRatio number of phases, but other ways, e.g. based on other decision criteria, will be evident to those skilled in the art.

The combined signal can then be upsampled back to the original sampling rate (step 1143).

In an alternative approach to steps 1141 and 1143, each of the filtered phase signals acc_poly_filt[p,k] obtained in step 1139 can be upsampled back to the original sampling rate to give acc_poly_filt_up[p,t] first (step 1145) and then the upsampled signals can be combined (step 1147). This approach has the benefit that the signals acc_poly_filt_up[p,t] can include the phase, and their combining accounts for the phase, i.e. for the integer t such that t = p + subRatio*k, acc_poly_filt_up[p,t] is set to acc_poly_filt[p,k]. The remaining samples are found using an interpolation filter (the same as the low-pass filter applied before the downsampling step; preferably, the remaining samples are set to the neighbouring assigned sample with "integer t", i.e. using a sample-and-hold scheme).

After step 1143 or 1149, a further median filter can be applied to the combined upsampled signal in order to remove any 'ringing' effects introduced by the interpolation filter. This median filter can have the same window size in seconds as the median filter applied in step 1139.

It will be appreciated that not all steps are mandatory. The core of the subMedian filter is the application of the non-linear filter at a downsampled rate. The anti-aliasing filter, as well as accounting for the different phases, are refinements that can be applied depending on the application at hand.

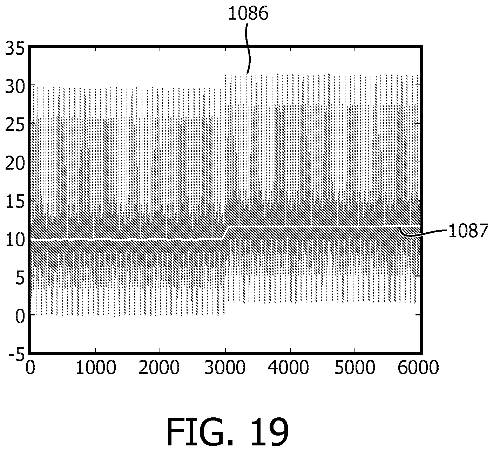

Fig. 19 is a graph illustrating the result of applying a subMedian filter to a signal 1086 comprising a set of four sinusoidal signals having different frequencies, phases and amplitudes and Gaussian noise. Furthermore, there is a step in the signal at time t = 3000. Line 1087 shows the result of applying a subMedian filter 1070 as described above to signal 1086, and it can be seen that the output provides a good estimate of the DC component and adequately tracks the step.

Adaptive Median Filter

As described above, in a preferred implementation, a filter, termed herein as an 'adaptive median' filter, is applied to the estimate of the vertical velocity output by integration block 1028 in order to estimate the offset and drift in that signal.

The purpose of the filter is to extract the velocity component in the velocity estimate that corresponds to the pulse form that deviates from a drifting DC (i.e. offset and drift) component. The offset may change and there may also be some drift.

During such a change, it is desirable to keep the current value for the offset, since it has been found that following the change in offset tends to follow the actual velocity signal and therefore leads to an underestimate in the actual velocity.

Therefore, in the adaptive median filter, the window size used to determine the median for a particular sample is adapted depending on the rank order of the median value in subwindows within the window. The rank order between the median values indicates whether there is an increase or decrease in the offset, and the subsequent adaptation (namely the choice of window size) aims to compensate for or prevent the underestimation in the value of the offset.

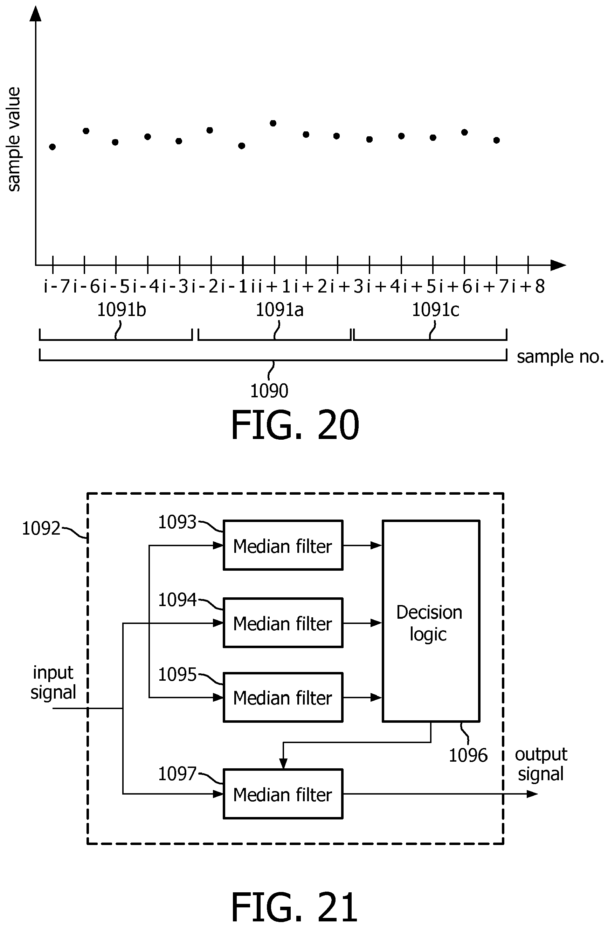

Fig. 20 shows part of a signal comprising 16 samples and a median filter window 1090 centred on the current sample i with a half-window size of 7. In the adaptive median filter, the median filter window 1090 is divided into three subwindows 1091a, 1091b and 1091c (with a half-window size of 2 in this example). One subwindow (1091a in Fig. 20) is centred on the current sample i, another subwindow (1091b in Fig. 20) is centred on sample i-5 (i.e. the current sample minus the full subwindow width), and the third subwindow (1091c in Fig. 20) is centred on sample i+5 (i.e. the current sample plus the full subwindow width).

As indicated above, the median value is calculated for each of the three subwindows and the three median values are ranked. The order of the ranking is used to determine the choice of window to use in producing the output of the adaptive median filter.

According to one exemplary implementation, the rule set used to determine the choice of window for calculating the output is as follows:

- if the median value of the centre subwindow 1091a is the maximum of the three median values, the output of the adaptive median filter is the median value in a subwindow having a half-window size that is larger than (e.g. twice) that of subwindow 1091a and that is centred on sample i;
- if the median value of the centre subwindow 1091a is between the values for the other subwindows 1091b and 1091c, the output of the adaptive median filter is the median value over a subwindow corresponding to the centre subwindow 1091a and the subwindow 1091b or 1091c that generated the highest median value; and
- if the median value of the centre subwindow 1091a is the minimum of the three median values, the output of the adaptive median filter is the median value obtained using the centre subwindow 1091a.

According to another, preferred, embodiment, the rule set used to determine the choice of window for calculating the output is as follows:

- if the median value of the centre subwindow 1091a is the maximum or minimum of the three median values, the output of the adaptive median filter is the median value in a subwindow having a half-window size that is larger than that of subwindow 1091a and that is centred on sample i;
- otherwise, the output of the adaptive median filter is the value used for the previous sample (i.e. i-1).

In one implementation, where the median value of the centre subwindow 1091a is the maximum or minimum of the three median values, a half-window size that is twice that of subwindow 1091a is used to generate the output value. It will be appreciated, however, that other sized windows, larger than that of subwindow 1091a, may be used.

Alternative actions to the use of the value for the previous sample where the median value of the centre subwindow 1091a is not the maximum or minimum of the three median values can include using the value from the current centre subwindow 1091a, or applying a recursive median filter to the current centre subwindow 1091a or on the whole window.

In this way, the use of a larger window when the centre subwindow provides the maximum or minimum median value reduces the effect that the median filter follows a rising/falling trend of short duration present in the velocity signal. Using the previous value when the centre subwindow does not provide the maximum or minimum median value provides the constancy discussed above.

Fig. 21 is a block diagram of an exemplary adaptive median filter 1092.

The input signal is provided to three filters, labelled 1093, 1094 and 1095, which apply a respective one of the three subwindows 1091a, 1091b and 1091c discussed above. In this embodiment, the filters are median filters, but it will be appreciated that the filters can be variants of a normal median filter. The output of each of these median filters is provided to decision logic 1096 which implements one of the rule sets described above and determines the window size to be used to generate the output of the adaptive median filter 1092.

A control signal indicating the required window size is output by the decision logic 1096 to a median filter 1097. Median filter 1097 also receives the input signal (with a suitable delay to allow for the processing delay introduced by median filters 1093, 1094 and 1095 and the decision logic 1096) and operates on the input signal using the required window size. The output of median filter 1097 is the output of the adaptive median filter.

It will be appreciated by those skilled in the art that the adaptive median filter 1092 can be implemented using an alternative arrangement of components to that shown in Fig. 21. For example, as the rule set may provide that the median value of the centre subwindow 1091a can be used as the output, the decision logic 1096 may simply output this value rather than require median filter 1097 recalculate it. Also, as is clear, reusing the previously computed output value doesn't require the full recomputation by the median filter 1097.

In addition, it will be appreciated that it is possible for the window to be divided into more than three subwindows, as required, and it is also possible for the subwindows to have different sizes.

**Claims**

1. A bed exit monitoring apparatus for monitoring a user and determining when the user has got out of a bed, the apparatus comprising:

   a processor that is configured to:

   receive measurements of the acceleration in three dimensions acting on a device that is attached to the user;
   perform a calibration procedure in which reference vectors corresponding to axes of a reference frame of the user are determined; and
   process the measurements to determine if the user has got out of bed by:

   determining a change in height of the device over time from the measurements of acceleration and/or from measurements of air pressure at the device;
   estimating the posture of the user over time from the measurements of the acceleration by determining a measure of the correspondence in direction of acceleration vectors derived from the measurements of acceleration and the one or more reference vectors corresponding to axes of the reference frame of the user, wherein the absolute value or magnitude of the measure of the correspondence in direction of the acceleration vectors and each reference vector indicates the correspondence of the measurements of acceleration to a particular posture of the user; and
   determining that the user has got out of bed if there is a change in height greater than a threshold that occurs between an estimated lying or sitting posture and an estimated upright posture;

   wherein the processor is configured to perform the calibration procedure by:

   searching a set of acceleration vectors derived from the measurements of acceleration for periods of time in which a particular component of acceleration exceeds a threshold to identify a first period of time in which the user is in a first posture and a second period of time in which the user is in a second, different, posture;
   averaging the acceleration vectors in each of the first and second periods of time to give respective first and second averaged vectors for each of the first and second periods of time, the first and second vectors being considered to be reference vectors for first and second axes of the reference frame of the user respectively; and
   determining a third vector for use as a reference vector for a third axis of the reference frame of the user as a vector that is orthogonal to the first and second vector;

   wherein the processor is further configured to set the value of the threshold to the average value of the particular component in a previously-identified period of time in which the particular component of acceleration exceeded the threshold.

2. A bed exit monitoring apparatus as claimed in claim 1, wherein
   the reference vectors correspond respectively to: the z-axis of the reference frame of the user which is in the posterior-anterior direction, the y-axis of the reference frame of the user which is vertical from bottom to top, and the x-axis of the reference frame of the user which is in the medial to lateral direction;
   the measure of the correspondence in direction of the acceleration vectors and the reference vector for the z-axis of the reference frame of the user indicates the correspondence of the measurements of acceleration to a prone or supine posture of the user;
   the measure of the correspondence in direction of the acceleration vectors and the reference vector for the y-axis of the reference frame of the user indicates the correspondence of the measurements of acceleration to an upright posture of the user; and
   the measure of the correspondence in direction of the acceleration vectors and the reference vector for the x-axis of the reference frame of the user indicates the correspondence of the measurements of acceleration to a posture in which the user is lying on their side.

3. A bed exit monitoring apparatus as claimed in claim 1 or 2, wherein the processor is configured to estimate the posture of the user for an acceleration vector based on the measure of the correspondence in direction that has the largest absolute value or magnitude.

4. A device that is configured to be worn by a user, the device comprising:

> an accelerometer that measures the acceleration acting on the device in three-dimensions; and
> an apparatus as claimed in any of claims 1 to 3.

5. A bed exit monitoring system, the system comprising:

> a device that is configured to be worn by a user, the device comprising an accelerometer that measures the acceleration acting on the device in three-dimensions; and
> a base unit that is configured to communicate with the device, and that comprises an apparatus as claimed in any of claims 1 to 3.

6. A method of monitoring a user and determining when the user has got out of a bed, the method comprising:

> measuring the acceleration in three dimensions that is acting on a device that is attached to the user;
> performing a calibration procedure in which reference vectors corresponding to axes of a reference frame of the user are determined; and
> processing the measurements to determine if the user has got out of bed by:

>> determining a change in height of the device over time from the measurements of acceleration and/or from measurements of air pressure at the device;
>> estimating the posture of the user over time from the measurements of the acceleration by determining a measure of the correspondence in direction of acceleration vectors derived from the measurements of acceleration and the one or more reference vectors corresponding to axes of the reference frame of the user, wherein the absolute value or magnitude of the measure of the correspondence in direction of the acceleration vectors and each reference vector indicate the correspondence of the measurements of acceleration to a particular posture of the user; and
>> determining that the user has got out of bed if there is a change in height greater than a threshold that occurs between an estimated lying or sitting posture and an estimated upright posture;

> wherein the step of performing a calibration procedure comprises:

>> searching a set of acceleration vectors derived from the measurements of acceleration for periods of time in which a particular component of acceleration exceeds a threshold to identify a first period of time in which the user is in a first posture and a second period of time in which the user is in a second, different, posture;
>> averaging the acceleration vectors in each of the first and second periods of time to give respective first and second averaged vectors for each of the first and second periods of time, the first and second vectors being considered to be reference vectors for first and second axes of the reference frame of the user respectively;
>> determining a third vector for use as a reference vector for a third axis of the reference frame of the user as a vector that is orthogonal to the first and second vector; and
>> setting the value of the threshold to the average value of the particular component in a previously-identified period of time in which the particular component of acceleration exceeded the threshold.

7. A method as claimed in claim 6, wherein
the reference vectors can correspond respectively to: the z-axis of the reference frame of the user which is in the posterior-anterior direction, the y-axis of the reference frame of the user which is vertical from bottom to top, and the x-axis of the reference frame of the user which is in the medial to lateral direction;
the measure of the correspondence in direction of the acceleration vectors and the reference vector for the z-axis of the reference frame of the user can indicate the correspondence of the measurements of acceleration to a prone or supine posture of the user;

the measure of the correspondence in direction of the acceleration vectors and the reference vector for the y-axis of the reference frame of the user can indicate the correspondence of the measurements of acceleration to an upright posture of the user; and

the measure of the correspondence in direction of the acceleration vectors and the reference vector for the x-axis of the reference frame of the user can indicate the correspondence of the measurements of acceleration to a posture in which the user is lying on their side.

**8.** A method as claimed in claim 6 or 7, wherein the step of processing measurements comprises estimating the posture of the user for an acceleration vector based on the measure of the correspondence in direction that has the largest absolute value or magnitude.

**9.** A computer program product, comprising a computer readable medium having computer program code embodied therein, the computer program code being configured such that, upon execution by a computer or processor, the computer or processor performs the method as claimed in any of claims 6 to 8.

**Patentansprüche**

**1.** Überwachungsvorrichtung für das Verlassen des Bettes zum Überwachen eines Benutzers und zum Bestimmen, wenn der Benutzer vom Bett aufgestanden ist, wobei die Vorrichtung umfasst:

einen Prozessor, der konfiguriert ist zum:

Empfangen von Messungen der Beschleunigung in drei Dimensionen, die auf ein Gerät einwirkt, das an dem Benutzer befestigt ist;
Durchführen eines Kalibriervorgangs, bei dem Referenzvektoren entsprechend den Achsen eines Referenzrahmens des Benutzers bestimmt werden; und
Verarbeiten der Messungen um zu bestimmen, ob der Benutzer vom Bett aufgestanden ist, durch:

Bestimmen einer Veränderung in der Höhe des Geräts im Zeitverlauf aus den Messungen der Beschleunigung und/oder aus Messungen des Luftdrucks an dem Gerät;
Schätzen der Haltung des Benutzers im Zeitverlauf aus den Messungen der Beschleunigung durch Bestimmen einer Messung der Richtungsübereinstimmung von Beschleunigungsvektoren, abgeleitet von den Messungen der Beschleunigung, und dem einen oder den mehreren Referenzvektoren entsprechend Achsen des Referenzrahmens des Benutzers, wobei der Absolutwert oder die Größe der Messung der Richtungsübereinstimmung der Beschleunigungsvektoren und jedes Referenzvektors die Übereinstimmung der Messungen der Beschleunigung mit einer speziellen Haltung des Benutzers angibt; und
Bestimmen, dass der Benutzer vom Bett aufgestanden ist, wenn es eine Veränderung in der Höhe, größer als ein Grenzwert, gibt, die auftritt zwischen einer geschätzten liegenden oder sitzenden Haltung und einer geschätzten aufrechten Haltung;

wobei der Prozessor konfiguriert ist, den Kalibriervorgang durchzuführen, durch:

Suchen eines Beschleunigungsvektorsatzes, abgeleitet von den Messungen der Beschleunigung, für Zeitdauern, in denen eine spezielle Beschleunigungskomponente einen Grenzwert übersteigt, um eine erste Zeitdauer zu identifizieren, in der der Benutzer in einer ersten Haltung ist, und eine zweite Zeitdauer, in der der Benutzer in einer zweiten, unterschiedlichen Haltung ist;
Mitteln der Beschleunigungsvektoren in jeder der ersten und zweiten Zeitdauer, um jeweilige erste und zweite gemittelte Vektoren für jede der ersten und zweiten Zeitdauer zu ergeben, wobei die ersten und zweiten Vektoren als Referenzvektoren jeweils für erste und zweite Achsen des Referenzrahmens des Benutzers gelten; und
Bestimmen eines dritten Vektors zur Verwendung als Referenzvektor für eine dritte Achse des Referenzrahmens des Benutzers als Vektor, der orthogonal zu dem ersten und zweiten Vektor ist;

wobei der Prozessor weiter konfiguriert ist, den Wert des Grenzwertes auf den Mittelwert der speziellen Komponente in einer zuvor identifizierten Zeitdauer zu setzen, in der die spezielle Beschleunigungskomponente den Grenzwert überstiegen hat.

**2.** Überwachungsvorrichtung für das Verlassen des Bettes nach Anspruch 1, wobei
die Referenzvektoren jeweils entsprechen: der Z-Achse des Referenzrahmens des Benutzers, die in der posterior-anterioren Richtung verläuft, der Y-Achse des Referenzrahmens des Benutzers, die vertikal von unten nach oben verläuft, und der X-Achse des Referenzrahmens des Benutzers, die in der medial-lateralen Richtung verläuft;
die Messung der Richtungsübereinstimmung der Beschleunigungsvektoren und des Referenzvektors für die Z-Achse des Referenzrahmens des Benutzers die Übereinstimmung der Beschleunigungsmessungen mit einer Haltung des Benutzers in Bauch- oder Rückenlage angibt;
die Messung der Richtungsübereinstimmung der Beschleunigungsvektoren und des Referenzvektors für die Y-Achse des Referenzrahmens des Benutzers die Übereinstimmung der Beschleunigungsmessungen mit einer aufrechten Haltung des Benutzers angibt; und
die Messung der Richtungsübereinstimmung der Beschleunigungsvektoren und des Referenzvektors für die X-Achse des Referenzrahmens des Benutzers die Übereinstimmung der Beschleunigungsmessungen mit einer Haltung angibt, in der der Benutzer auf der Seite liegt.

**3.** Überwachungsvorrichtung für das Verlassen des Bettes nach Anspruch 1 oder 2, wobei der Prozessor konfiguriert ist zum Schätzen der Haltung des Benutzers für einen Beschleunigungsvektor basierend auf der Messung der Richtungsübereinstimmung, die den größten Absolutwert oder die größte Größe aufweist.

**4.** Gerät, das konfiguriert ist, von einem Benutzer getragen zu werden, wobei das Gerät umfasst:

einen Beschleunigungsmesser, der die Beschleunigung misst, die auf das Gerät in drei Dimensionen einwirkt; und
eine Vorrichtung nach einem der Ansprüche 1 bis 3.

**5.** Überwachungssystem für das Verlassen des Bettes, wobei das System umfasst:

ein Gerät, das konfiguriert ist, von einem Benutzer getragen zu werden, wobei das Gerät einen Beschleunigungsmesser umfasst, der die Beschleunigung misst, die auf das Gerät in drei Dimensionen einwirkt; und
eine Basiseinheit, die konfiguriert ist, mit dem Gerät zu kommunizieren, und die eine Vorrichtung nach einem der Ansprüche 1 bis 3 umfasst.

**6.** Verfahren zum Überwachen eines Benutzers und zum Bestimmen, wenn der Benutzer vom Bett aufgestanden ist, wobei das Verfahren umfasst:

Messen der Beschleunigung in drei Dimensionen, die auf ein Gerät einwirkt, das an dem Benutzer befestigt ist;
Durchführen eines Kalibriervorgangs, bei dem Referenzvektoren entsprechend den Achsen eines Referenzrahmens des Benutzers bestimmt werden; und
Verarbeiten der Messungen um zu bestimmen, ob der Benutzer vom Bett aufgestanden ist, durch:

Bestimmen einer Veränderung in der Höhe des Geräts im Zeitverlauf aus den Messungen der Beschleunigung und/oder aus Messungen des Luftdrucks an dem Gerät;
Schätzen der Haltung des Benutzers im Zeitverlauf aus den Messungen der Beschleunigung durch Bestimmen einer Messung der Richtungsübereinstimmung von Beschleunigungsvektoren, abgeleitet von den Messungen der Beschleunigung, und dem einen oder den mehreren Referenzvektoren entsprechend Achsen des Referenzrahmens des Benutzers, wobei der Absolutwert oder die Größe der Messung der Richtungsübereinstimmung der Beschleunigungsvektoren und jedes Referenzvektors die Übereinstimmung der Messungen der Beschleunigung mit einer speziellen Haltung des Benutzers angibt; und
Bestimmen, dass der Benutzer vom Bett aufgestanden ist, wenn es eine Veränderung in der Höhe, größer als ein Grenzwert, gibt, die auftritt zwischen einer geschätzten liegenden oder sitzenden Haltung und einer geschätzten aufrechten Haltung;

wobei der Schritt des Durchführens eines Kalibriervorgangs umfasst:

Suchen eines Beschleunigungsvektorsatzes, abgeleitet von den Messungen der Beschleunigung, für Zeitdauern, in denen eine spezielle Beschleunigungskomponente einen Grenzwert übersteigt, um eine erste Zeitdauer zu identifizieren, in der der Benutzer in einer ersten Haltung ist, und eine zweite Zeitdauer, in der der Benutzer in einer zweiten, unterschiedlichen Haltung ist;
Mitteln der Beschleunigungsvektoren in jeder der ersten und zweiten Zeitdauer, um jeweilige erste und zweite gemittelte Vektoren für jede der ersten und zweiten Zeitdauer zu ergeben, wobei die ersten und zweiten Vektoren als Referenzvektoren jeweils für erste und zweite Achsen des Referenzrahmens des Benutzers gelten;

Bestimmen eines dritten Vektors zur Verwendung als Referenzvektor für eine dritte Achse des Referenzrahmens des Benutzers als Vektor, der orthogonal zu dem ersten und zweiten Vektor ist; und

Setzen des Wertes des Grenzwertes auf den Mittelwert der speziellen Komponente in einer zuvor identifizierten Zeitdauer, in der die spezielle Beschleunigungskomponente den Grenzwert überstiegen hat.

**7.** Verfahren nach Anspruch 6, wobei

die Referenzvektoren jeweils entsprechen können: der Z-Achse des Referenzrahmens des Benutzers, die in der posterior-anterioren Richtung verläuft, der Y-Achse des Referenzrahmens des Benutzers, die vertikal von unten nach oben verläuft, und der X-Achse des Referenzrahmens des Benutzers, die in der medial-lateralen Richtung verläuft;

die Messung der Richtungsübereinstimmung der Beschleunigungsvektoren und des Referenzvektors für die Z-Achse des Referenzrahmens des Benutzers die Übereinstimmung der Beschleunigungsmessungen mit einer Haltung des Benutzers in Bauch- oder Rückenlage angeben kann;

die Messung der Richtungsübereinstimmung der Beschleunigungsvektoren und des Referenzvektors für die Y-Achse des Referenzrahmens des Benutzers die Übereinstimmung der Beschleunigungsmessungen mit einer aufrechten Haltung des Benutzers angeben kann; und

die Messung der Richtungsübereinstimmung der Beschleunigungsvektoren und des Referenzvektors für die X-Achse des Referenzrahmens des Benutzers die Übereinstimmung der Beschleunigungsmessungen mit einer Haltung angeben kann, in der der Benutzer auf der Seite liegt.

**8.** Verfahren nach Anspruch 6 oder 7, wobei der Schritt des Verarbeitens von Messungen das Schätzen der Haltung des Benutzers für einen Beschleunigungsvektor basierend auf der Messung der Richtungsübereinstimmung, die den größten Absolutwert oder die größte Größe aufweist, umfasst.

**9.** Computerprogrammprodukt, umfassend ein computerlesbares Medium mit einem darin eingebetteten Computerprogrammcode, wobei der Computerprogrammcode so konfiguriert ist, dass, nach Ausführung durch einen Computer oder Prozessor, der Computer oder Prozessor das Verfahren nach einem der Ansprüche 6 bis 8 durchführt.

**Revendications**

**1.** Appareil de surveillance de sortie de lit pour surveiller un utilisateur et déterminer lorsque l'utilisateur est sorti d'un lit, l'appareil comprenant :

un processeur qui est configuré pour :

recevoir des mesures de l'accélération en trois dimensions agissant sur un dispositif qui est attaché à l'utilisateur ;

réaliser une procédure d'étalonnage dans laquelle des vecteurs de référence correspondant aux axes d'un cadre de référence de l'utilisateur sont déterminés ; et

traiter les mesures pour déterminer si l'utilisateur est sorti du lit en :

déterminant un changement de hauteur du dispositif avec le temps à partir des mesures d'accélération et/ou des mesures de pression d'air au niveau du dispositif ;

estimant la posture de l'utilisateur avec le temps à partir des mesures de l'accélération en déterminant une mesure de la correspondance en direction de vecteurs d'accélération dérivée des mesures d'accélération et du ou des vecteurs de référence correspondant aux axes du cadre de référence de l'utilisateur, dans lequel la valeur ou grandeur absolue de la mesure de la correspondance en direction des vecteurs d'accélération et de chaque vecteur de référence indique la correspondance des mesures d'accélération avec une posture particulière de l'utilisateur ; et

déterminant que l'utilisateur est sorti du lit en présence d'un changement de hauteur supérieur à un seuil qui se produit entre une posture allongée ou assise estimée et une position debout estimée ;

dans lequel le processeur est configuré pour effectuer la procédure d'étalonnage en :

recherchant un ensemble de vecteurs d'accélération dérivés des mesures d'accélération pendant des périodes de temps pendant lesquelles une composante d'accélération particulière dépasse un seuil pour identifier une première période de temps pendant laquelle l'utilisateur est dans une première posture et une seconde période de temps pendant laquelle l'utilisateur est dans une seconde posture différente ;
établissant la moyenne les vecteurs d'accélération dans chacune des première et seconde périodes de temps pour donner des premier et deuxième vecteurs moyennés respectifs pour chacune des première et seconde périodes de temps, les premier et second vecteurs étant considérés comme étant des vecteurs de référence pour les premier et deuxième axes du cadre de référence de l'utilisateur respectivement ; et
déterminant un troisième vecteur pour utilisation en tant que vecteur de référence pour un troisième axe du cadre de référence de l'utilisateur en tant que vecteur qui est orthogonal au premier et au deuxième vecteur ;

dans lequel le processeur est en outre configuré pour définir la valeur du seuil à la valeur moyenne de la composante particulière dans une période de temps préalablement identifiée pendant laquelle la composante d'accélération particulière a dépassé le seuil.

2. Appareil de surveillance de sortie de lit selon la revendication 1, dans lequel
les vecteurs de référence correspondent respectivement à : l'axe z du cadre de référence de l'utilisateur qui est dans la direction postérieure-antérieure, l'axe y du cadre de référence de l'utilisateur qui est vertical de bas en haut, et l'axe x du cadre de référence de l'utilisateur qui est dans la direction médiale à latérale ;
la mesure de la correspondance en direction des vecteurs d'accélération et du vecteur de référence pour l'axe z du cadre de référence de l'utilisateur indique la correspondance des mesures d'accélération avec une posture couché sur le ventre ou couché sur le dos de l'utilisateur ;
la mesure de la correspondance en direction des vecteurs d'accélération et du vecteur de référence pour l'axe y du cadre de référence de l'utilisateur indique la correspondance des mesures d'accélération avec une posture debout de l'utilisateur ; et
la mesure de la correspondance en direction des vecteurs d'accélération et du vecteur de référence de l'axe x du cadre de référence de l'utilisateur indique la correspondance des mesures d'accélération avec une posture dans laquelle l'utilisateur est allongé sur le côté.

3. Appareil de surveillance de sortie de lit selon la revendication 1 ou 2, dans lequel le processeur est configuré pour estimer la posture de l'utilisateur pour un vecteur d'accélération basé sur la mesure de la correspondance en direction qui a la valeur ou grandeur absolue la plus élevée.

4. Dispositif qui est configuré pour être porté par un utilisateur, le dispositif comprenant :

un accéléromètre qui mesure l'accélération agissant sur le dispositif en trois dimensions ; et
un appareil selon l'une quelconque des revendications 1 à 3.

5. Système de surveillance de sortie de lit, le système comprenant :

un dispositif qui est configuré pour être porté par un utilisateur, le dispositif comprenant un accéléromètre qui mesure l'accélération agissant sur le dispositif en trois dimensions ; et
une unité de base qui est configurée pour communiquer avec le dispositif, et qui comprend un appareil selon l'une quelconque des revendications 1 à 3.

6. Procédé de surveillance d'un utilisateur et de détermination du moment où l'utilisateur est sorti d'un lit, le procédé comprenant :

la mesure de l'accélération en trois dimensions qui agit sur un dispositif qui est attaché à l'utilisateur ;
la réalisation d'une procédure d'étalonnage dans laquelle des vecteurs de référence correspondant aux axes d'un cadre de référence de l'utilisateur sont déterminés ; et
le traitement des mesures pour déterminer si l'utilisateur est sorti du lit en ;
déterminant un changement de hauteur du dispositif avec le temps à partir des mesures d'accélération et/ou des mesures de pression d'air au niveau du dispositif ;
estimant la posture de l'utilisateur avec le temps à partir des mesures de l'accélération en déterminant une mesure de la correspondance en direction de vecteurs d'accélération dérivée des mesures d'accélération et du ou des vecteurs de référence correspondant aux axes du cadre de référence de l'utilisateur, dans lequel la valeur ou grandeur absolue de la mesure de la correspondance en direction des vecteurs d'accélération et de chaque vecteur de référence indique la correspondance des mesures d'accélération avec une posture particulière de l'utilisateur ; et

déterminant que l'utilisateur est sorti du lit en présence d'un changement de hauteur supérieur à un seuil qui se produit entre une posture allongée ou assise estimée et une position debout estimée ;

dans lequel l'étape de réalisation d'une procédure d'étalonnage comprend :

la recherche d'un ensemble de vecteurs d'accélération dérivés des mesures d'accélération pendant des périodes de temps pendant lesquelles une composante d'accélération particulière dépasse un seuil pour identifier une première période de temps pendant laquelle l'utilisateur est dans une première posture et une seconde période de temps pendant laquelle l'utilisateur est dans une seconde posture différente ;

l'établissement de la moyenne des vecteurs d'accélération dans chacune des première et seconde périodes de temps pour donner des premier et deuxième vecteurs moyennés respectifs pour chacune des première et seconde périodes de temps, les premier et deuxième vecteurs étant considérés comme étant des vecteurs de référence pour les premier et deuxième axes du cadre de référence de l'utilisateur respectivement ;

la détermination d'un troisième vecteur pour utilisation en tant que vecteur de référence pour un troisième axe du cadre de référence de l'utilisateur en tant que vecteur qui est orthogonal au premier et au deuxième vecteur ; et

la définition de la valeur du seuil à la valeur moyenne de la composante particulière dans une période de temps préalablement identifiée pendant laquelle la composante d'accélération particulière a dépassé le seuil.

7. Procédé selon la revendication 6, dans lequel

les vecteurs de référence peuvent correspondent respectivement à : l'axe z du cadre de référence de l'utilisateur qui est dans la direction postérieure-antérieure, l'axe y du cadre de référence de l'utilisateur qui est vertical de bas en haut, et l'axe x du cadre de référence de l'utilisateur qui est dans la direction médiale à latérale ;

la mesure de la correspondance en direction des vecteurs d'accélération et du vecteur de référence pour l'axe z du cadre de référence de l'utilisateur peut indiquer la correspondance des mesures d'accélération avec une posture couché sur le ventre ou couché sur le dos de l'utilisateur ;

la mesure de la correspondance en direction des vecteurs d'accélération et du vecteur de référence pour l'axe y du cadre de référence de l'utilisateur peut indiquer la correspondance des mesures d'accélération avec une posture debout de l'utilisateur ; et

la mesure de la correspondance en direction des vecteurs d'accélération et du vecteur de référence de l'axe x du cadre de référence de l'utilisateur peut indiquer la correspondance des mesures d'accélération avec une posture dans laquelle l'utilisateur est allongé sur le côté.

8. Procédé selon la revendication 6 ou 7, dans lequel l'étape de traitement des mesures comprend l'estimation de la posture de l'utilisateur pour un vecteur d'accélération basé sur la mesure de la correspondance en direction qui a la valeur ou grandeur absolue la plus élevée.

9. Produit de programme informatique, comprenant un support lisible par ordinateur ayant un code de programme informatique intégré à celui-ci, le code de programme informatique étant configuré de telle sorte que, lorsqu'il est exécuté par un ordinateur ou processeur, l'ordinateur ou processeur réalise le procédé selon l'une quelconque des revendications 6 à 8.

FIG. 1

(a)

(b)

FIG. 2

EP 2 741 669 B1

Position in global coordinate system

FIG. 3

Barometer

FIG. 4

101 — Calibrate device

103 — Obtain measurements of acceleration acting on a device attached to a user

Estimate the height or change in height of the device over time

Estimate the posture of the user over time

105

109

Does estimated height or change in height exceed a threshold value?

No

Yes

107

Do estimated change in height and postures occur in a temporal order consistent with a bed exit?

No

111

Yes

113 — Bed exit/bed exit attempt detected

115 — Trigger alarm

FIG. 5

**FIG. 6**

**FIG. 7**

FIG. 8

EP 2 741 669 B1

153

Await next sample
for which change
in height exceeds
the threshold

Yes

151

Is there
a period of supine or
side posture in a time window
after the current
sample, k?

No

155

No

Is there
a period of supine or
side posture in a time window
before the current
sample, k?

Yes

157

No

Is there a
period of upright
posture in a time window
after the current
sample, k?

Yes, bed exit

FIG. 9

171 — Set "inBed" to false

173 — Is the posture for the current sample k supine or lying on their side?

175 — Retrieve next sample ← No

Yes ↓

177 — Set "inBed" to true

181 — Retrieve next sample

179 — Is change in height at the current sample k greater than the threshold?
No

Yes ↓

183 — Is there a period of supine or side posture in a time window after the current sample, k?
Yes

No ↓

185 — Is there a period of upright posture in a time window after the current sample, k?
No

Yes ↓

187 — Bed exit detected – set "inBed" to false

# FIG. 10

FIG. 11

1101 — Measure accelerations acting on the fall detector/user in three dimensions

1103 — Estimate the acceleration, $acc_z$, acting in the vertical direction

1105 — Filter the vertical acceleration to estimate the component of acceleration due to gravity in the vertical acceleration

1107 — Subtract the gravity component from the vertical acceleration to give the vertical acceleration due to motion (i.e. non-gravitational)

1109 — Integrate the vertical acceleration due to motion to give vertical velocity

1111 — Filter the vertical velocity to estimate offset and drift components

1113 — Subtract the estimated offset and drift components from the vertical velocity to give a filtered vertical velocity

1115 — Integrate the corrected vertical velocity to give an estimate of the height of the fall detector/user

# FIG. 12

FIG. 13(a)

FIG. 13(b)

FIG. 13(c)

EP 2 741 669 B1

Velocity before velocity filter

FIG. 13(d)

FIG. 13(e)

FIG. 13(f)

FIG. 14

EP 2 741 669 B1

FIG. 15

FIG. 16

FIG. 17

1131 — Set
sub-sampling
ratio

1133 — Set cut-off
frequency of
low-pass filter

1135 — Low-pass filter the
input signal, acc,
to give acc_LPF

1137 — Downsample acc_LPF
into polyphase signal
acc_poly

1139 — Per phase, p, apply
median filter, giving
acc_poly_filt [p, k]

1141 — Combine obtained
filtered phase signals

Upsample each filtered
phase signal using
an interpolation filter — 1145

1143 — Upsample combined
signal using an
interpolation filter

Combine the
upsampled signal — 1147

1149 — Apply a median filter
to the combined
upsampled signal

FIG. 18

FIG. 19

FIG. 20

FIG. 21

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010134010 A
- EP 1115350 A
- WO 2013024461 A1
- EP 61524813 A
- US 61524813 B
- WO 20100035191 A